# EUROPEAN PATENT APPLICATION

(11) **EP 4 624 463 A1**
(43) Date of publication of application: **01.10.2025**
(21) Application number: 23892875.8
(22) Date of filing: 02.02.2023
(51) Int. Cl.: C07D 401/04, C07D 401/14, C07D 413/14, A61P 35/00, A61K 31/506

(54) **SPIRO DERIVATIVE AS KIF18A INHIBITOR**

(30) Priority: 25.11.2022 CN 202211486927
(71) Applicant: Insilico Medicine IP Limited, Central, Hong Kong (HK)
(72) Inventor: DING, Xiao, Shanghai 201208 (CN); REN, Feng, Shanghai 201208 (CN); WANG, Hongbin, Shanghai 201208 (CN); ZHENG, Min, Shanghai 201208 (CN); ZHU, Wei, Shanghai 201208 (CN)
(74) Representative: Mewburn Ellis LLP
(86) International application number: PCT/CN2023/074248
(87) International publication number: WO 2024/108765

(57) **Abstract**

The present disclosure discloses a class of spirocyclic derivatives as KIF18A inhibitors. More specifically, the present disclosure discloses a compound represented by Formula II, or a pharmaceutically acceptable salt thereof. The spirocyclic derivative has KIF 18A inhibitory activity and can be used for the treatment of cancer.

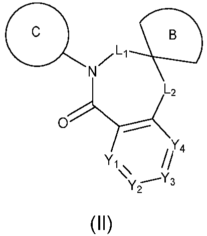

## Description

The present application claims priority to Chinese Patent Application No. 202211486927.1, filed on November 25, 2022, titled "A Class of Spirocyclic Derivatives as KIF18A Inhibitors", the contents of which are incorporated in entirety herein by citation.

### Technical Field

The present disclosure relates to the field of medicinal chemistry, and more specifically, to a class of spirocyclic derivatives as KIF18A inhibitors.

### Background Art

Cancer is one of the most prevalent diseases afflicting humankind and is one of the leading causes of death worldwide. It is typically characterized by unregulated cell proliferation. Damage to one or more genes responsible for cellular pathways (specifically those that regulate the progression of proliferation through cell cycle and centrosome cycle control) may lead to the loss of normal regulation of cell proliferation. These dysregulated genes may encode various tumor suppressors or oncogenic proteins, which participate in a cascade of events resulting in unchecked cell cycle progression and cell proliferation. Various kinases and kinesins have been identified as playing key roles in regulating and driving the cell cycle and mitosis in both normal and cancerous cells.

The KIF18A gene belongs to the kinesin-8 subfamily and is a plus-end-directed motor [protein]. KIF18A is believed to regulate the dynamics of the plus ends of kinetochore microtubules to control proper chromosome positioning and spindle tension. Depletion of human KIF18A in HeLa cervical cancer cells leads to longer spindles, increased chromosome oscillation during metaphase, and activation of the mitotic spindle assembly checkpoint (MI Mayr et al., Current Biology 17, 488-98, 2007). KIF18A appears to be a viable target for cancer therapy. KIF18A is overexpressed in various types of cancer, including but not limited to colon cancer, breast cancer, lung cancer, pancreatic cancer, prostate cancer, bladder cancer, head cancer, neck cancer, cervical cancer, and ovarian cancer. Moreover, in cancer cell lines, gene deletion, knockout, or inhibition of KIF18A affects the mitotic spindle apparatus. Specifically, inhibition of KIF18A has been found to induce mitotic cell arrest, a known vulnerability that can promote mitotic cell death through apoptosis, mitotic catastrophe, or polyploidy-driven lethality, or death following mitotic slippage during interphase.

Therefore, there is an urgent need to develop a new inhibitor of the KIF18A protein.

### Summary of the Invention

The technical problem to be solved by the present disclosure is the limited variety of existing KIF18A inhibitors. To address this, the present disclosure provides a class of spirocyclic derivatives as KIF18A inhibitors. These spirocyclic derivatives are a novel class of compounds with good inhibitory activity against KIF18A.

The present disclosure provides a compound represented by Formula II, or a pharmaceutically acceptable salt thereof, 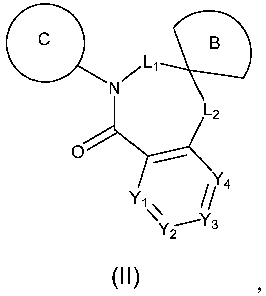
wherein ring C is selected from a C₆₋₁₂ aryl group, a 5- to 12-membered heteroaryl group, a C₃₋₁₈ cycloalkyl group, and a 3- to 18-membered heterocyclyl group, wherein the C₆₋₁₂ aryl group, 5- to 12-membered heteroaryl group, C₃₋₁₈ cycloalkyl group, or 3- to 18-membered heterocyclyl group is optionally substituted with 1, 2, 3, or 4 R_{c} groups;
R_{c} is independently selected from a halogen, a hydroxyl group, a cyano group, SF₅, -S(=O)₂R, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group, wherein the C₁₋₁₂ alkyl group, C₂₋₁₂ alkenyl group, C₂₋₁₂ alkynyl group, C₁₋₁₂ heteroalkyl group, C₂₋₁₂ heteroalkenyl group, C₂₋₁₂ heteroalkynyl group, C₆₋₁₂ aryl group, 5- to 12-membered heteroaryl group, C₃₋₁₂ cycloalkyl group, or 3- to 12-membered heterocyclyl group is optionally substituted with 1, 2, 3, or 4 R_{cc} groups;
alternatively, two R_{c} groups may be attached to the same carbon atom and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{cc} groups;
alternatively, two R_{c} groups may be attached to different carbon atoms and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{cc} groups;
L₁ is selected from a single bond, O, S, N(R_{L}), a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, and a C₂₋₁₂ heteroalkynyl group, wherein the N(R_{L}), C₁₋₁₂ alkyl group, C₂₋₁₂ alkenyl group, C₂₋₁₂ alkynyl group, C₁₋₁₂ heteroalkyl group, C₂₋₁₂ heteroalkenyl group, or C₂₋₁₂ heteroalkynyl group is optionally substituted with 1, 2, 3, or 4 R_{L1} groups;
L₂ is selected from a single bond, O, S, N(R_{L}), N(R_{L})C(=O), a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, and a C₂₋₁₂ heteroalkynyl group, wherein the N(R_{L}), N(R_{L})C(=O), C₁₋₁₂ alkyl group, C₂₋₁₂ alkenyl group, C₂₋₁₂ alkynyl group, C₁₋₁₂ heteroalkyl group, C₂₋₁₂ heteroalkenyl group, or C₂₋₁₂ heteroalkynyl group is optionally substituted with 1, 2, 3, or 4 R_{L2} groups;
ring B is selected from a C₃₋₁₂ cycloalkyl group and a 3- to 12-membered heterocyclyl group, wherein the C₃₋₁₂ cycloalkyl group or 3- to 12-membered heterocyclyl group is optionally substituted with 1, 2, 3, or 4 R_{b} groups;
R_{b} is independently selected from a halogen, a hydroxyl group, a cyano group, SF₅, a C₁₋₁₂ alkyl group, a C₂₋₁₂ alkenyl group, a C₂₋₁₂ alkynyl group, a C₂₋₁₂ heteroalkenyl group, a C₂₋₁₂ heteroalkynyl group, a C₁₋₁₂ heteroalkyl group, a C₆₋₁₂ aryl group, a 5- to 12-membered heteroaryl group, a C₃₋₁₂ cycloalkyl group, and a 3- to 12-membered heterocyclyl group;
alternatively, two R_{b} groups are attached to the same carbon atom to form =C(R_{bbb})₂ or =O;
alternatively, two R_{b} groups may be attached to the same carbon atom and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{bb} groups;
alternatively, two R_{b} groups may be attached to different carbon atoms and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{bb} groups;
Y₁ is selected from N and C(R_{Y1});
Y₂ is selected from N and C(R_{Y2});
Y₃ is selected from N and C(R_{Y3});
Y₄ is selected from N and C(R_{Y4});
R_{Y1}, R_{Y2}, R_{Y3}, and R_{Y4} are each independently selected from hydrogen, a halogen, a hydroxyl group, a cyano group, SF₅, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group, wherein the C₁₋₁₂ alkyl group, C₂₋₁₂ alkenyl group, C₂₋₁₂ alkynyl group, C₁₋₁₂ heteroalkyl group, C₂₋₁₂ heteroalkenyl group, C₂₋₁₂ heteroalkynyl group, C₆₋₁₂ aryl group, 5- to 12-membered heteroaryl group, C₃₋₁₂ cycloalkyl group, or 3- to 12-membered heterocyclyl group is optionally substituted with 1, 2, 3, or 4 R_{Y} groups;
alternatively, R_{Y1}, R_{Y2}, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{Y} groups;
alternatively, R_{Y2}, R_{Y3}, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{Y} groups;
alternatively, R_{Y3}, R_{Y4}, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{Y} groups;
R_{Y} is independently selected from a halogen, a hydroxyl group, a cyano group, SF₅, -S(=O)₂R, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group, wherein the C₁₋₁₂ alkyl group, C₂₋₁₂ alkenyl group, C₂₋₁₂ alkynyl group, C₁₋₁₂ heteroalkyl group, C₂₋₁₂ heteroalkenyl group, C₂₋₁₂ heteroalkynyl group, C₆₋₁₂ aryl group, 5- to 12-membered heteroaryl group, C₃₋₁₂ cycloalkyl group, or 3- to 12-membered heterocyclyl group is optionally substituted with 1, 2, 3, or 4 R' groups;
R is independently selected from a halogen, a hydroxyl group, a cyano group, SF₅, -S(=O)₂R, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group, wherein the C₁₋₁₂ alkyl group, C₂₋₁₂ alkenyl group, C₂₋₁₂ alkynyl group, C₁₋₁₂ heteroalkyl group, C₂₋₁₂ heteroalkenyl group, C₂₋₁₂ heteroalkynyl group, C₆₋₁₂ aryl group, 5- to 12-membered heteroaryl group, C₃₋₁₂ cycloalkyl group, or 3- to 12-membered heterocyclyl group is optionally substituted with 1, 2, 3, or 4 R' groups;
R_{bb}, R_{cc}, R_{L}, R_{L1}, R_{L2}, and R' are each independently selected from a halogen, a hydroxyl group, a cyano group, SF₅, -S(=O)₂R, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group;
alternatively, two R_{cc} groups are attached to the same carbon atom to form =C(R_{ccc})₂ or =O;
alternatively, two R' groups are attached to the same carbon atom to form =C(R")₂ or =O;
R_{bbb}, R_{ccc}, and R" are each independently selected from a halogen, a hydroxyl group, a cyano group, SF₅, a C₁₋₁₂ alkyl group, a C₂₋₁₂ alkenyl group, a C₂₋₁₂ alkynyl group, a C₂₋₁₂ heteroalkenyl group, a C₂₋₁₂ heteroalkynyl group, a C₁₋₁₂ heteroalkyl group, a C₆₋₁₂ aryl group, a 5- to 12-membered heteroaryl group, a C₃₋₁₂ cycloalkyl group, and a 3- to 12-membered heterocyclyl group;
the heteroalkyl group, heteroalkenyl group, heteroalkynyl group, heterocyclyl group, and heteroaryl group each comprise 1, 2, 3, or 4 heteroatoms or heteroatomic groups independently selected from O, NH, S, C(=O), C(=O)O, S(=O), S(=O)₂, S(=O)(=NH), and N.

In some embodiments, in the compound represented by Formula II or a pharmaceutically acceptable salt thereof, the definitions of certain groups are as described below, while the definitions of groups not mentioned herein are as described in any of the preceding embodiments (hereinafter referred to as "in some embodiments").

In some embodiments, ring C is selected from a phenyl group, a 5- to 6-membered monocyclic heteroaryl group, a saturated, partially saturated, or unsaturated fused bicyclic ring system composed of two 5- to 6-membered rings and comprising 0, 1, 2, or 3 N atoms and 0 or 1 atom selected from O and S, and a saturated, partially saturated, or unsaturated fused tricyclic ring system composed of three 5- to 6-membered rings and comprising 0, 1, 2, or 3 N atoms and 0 or 1 atom selected from O and S, wherein the phenyl group, the 5- to 6-membered monocyclic heteroaryl group, the fused bicyclic ring system composed of two 5- to 6-membered rings, and the fused tricyclic ring system composed of three 5- to 6-membered rings are each optionally substituted with 1, 2, 3, or 4 R_{c} groups.

In some embodiments of the present disclosure, ring C is selected from 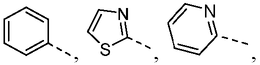 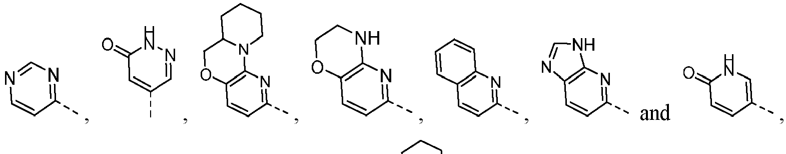 wherein 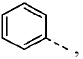 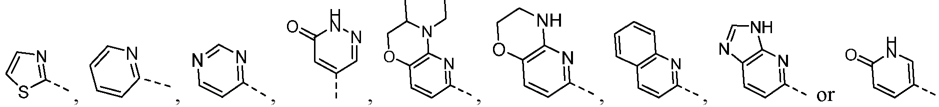 is optionally substituted by 1, 2, 3, or 4 R_{c} groups, and other variables are as defined herein.

In some embodiments of the present disclosure, R_{c} is independently selected from a halogen, a hydroxyl group, a cyano group, SF₅, -S(=O)₂R, a C₁₋₆ alkyl group, a C₂₋₆ alkenyl group, a C₂₋₆ alkynyl group, a C₁₋₆ heteroalkyl group, a C₂₋₆ heteroalkenyl group, a C₂₋₆ heteroalkynyl group, a phenyl group, a 5- to 6-membered heteroaryl group, a C₃₋₆ cycloalkyl group, and a 3- to 6-membered heterocyclyl group, wherein the C₁₋₆ alkyl group, C₂₋₆ alkenyl group, C₂₋₆ alkynyl group, C₁₋₆ heteroalkyl group, C₂₋₆ heteroalkenyl group, C₂₋₆ heteroalkynyl group, phenyl group, 5- to 6-membered heteroaryl group, C₃₋₆ cycloalkyl group, or 3- to 6-membered heterocyclyl group is optionally substituted with 1, 2, 3, or 4 R_{cc} groups, and other variables are as defined herein.

In some embodiments of the present disclosure, R_{c} is independently selected from a halogen, a hydroxyl group, a cyano group, SF₅, -S(=O)₂R, a C₁₋₃ alkyl group, a C₁₋₃ alkoxy group, a 6-membered heteroaryl group, and a C₄₋₆ cycloalkyl group, wherein the C₁₋₃ alkyl group or C₁₋₃ alkoxy group is optionally substituted with 1, 2, 3, or 4 R_{cc} groups, and other variables are as defined herein.

In some embodiments of the present disclosure, R_{cc} is independently selected from a halogen, a hydroxyl group, a cyano group, SF₅, -S(=O)₂R, a C₁₋₆ alkyl group, a C₂₋₆ alkenyl group, a C₂₋₆ alkynyl group, a C₁₋₆ heteroalkyl group, a C₂₋₆ heteroalkenyl group, a C₂₋₆ heteroalkynyl group, a phenyl group, a 5- to 6-membered heteroaryl group, a C₃₋₆ cycloalkyl group, and a 3- to 6-membered heterocyclyl group, and other variables are as defined herein.

In some embodiments of the present disclosure, R_{cc} is independently selected from a halogen, a hydroxyl group, a cyano group, SF₅, -S(=O)₂R, a C₁₋₃ alkyl group, a C₁₋₃ alkoxy group, a 6-membered heteroaryl group, and a C₄₋₆ cycloalkyl group; alternatively, two Rcc groups are attached to the same carbon atom to form =CF₂ or =O, and other variables are as defined herein.

In some embodiments of the present disclosure, ring C is selected from 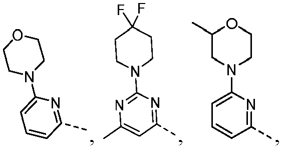 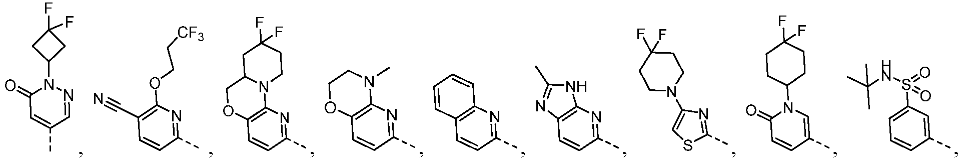 and 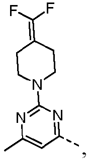 and other variables are as defined herein.

In some embodiments, ring B is selected from a C₃₋₆ cycloalkyl group and a 3- to 6-membered heterocyclyl group, wherein the C₃₋₆ cycloalkyl group or 3- to 6-membered heterocyclyl group is optionally substituted with 1, 2, 3, or 4 R_{b} groups, and other variables are as defined herein.

In some embodiments, ring B is selected from a C₃₋₆ cycloalkyl group and a 5- to 6-membered heterocycloalkyl group, wherein the C₃₋₆ cycloalkyl group or 5- to 6-membered heterocycloalkyl group is optionally substituted with 1, 2, 3, or 4 R_{b} groups, and other variables are as defined herein.

In some embodiments, R_{b} is independently selected from a halogen, a hydroxyl group, a cyano group, SF₅, a C₁₋₆ alkyl group, a C₂₋₆ alkenyl group, a C₂₋₆ alkynyl group, a C₁₋₆ heteroalkyl group, a C₂₋₆ heteroalkenyl group, a C₂₋₆ heteroalkynyl group, a phenyl group, a 5- to 9-membered heteroaryl group, a C₃₋₆ cycloalkyl group, and a 3- to 9-membered heterocyclyl group, and other variables are as defined herein.

In some embodiments, two R_{b} groups are attached to the same carbon atom to form =C(R_{bbb})₂ or =O, and other variables are as defined herein.

In some embodiments, two R_{b} groups are attached to the same carbon atom to form =C(F)₂ or =O, and other variables are as defined herein.

In some embodiments, two R_{b} groups may be attached to the same carbon atom and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{bb}groups, and other variables are as defined herein.

In some embodiments, two R_{b} groups may be attached to the same carbon atom and, together with the carbon to which they are attached, form a C₃₋₆ cycloalkyl ring or a 3- to 6-membered heterocyclic ring, wherein the C₃₋₆ cycloalkyl ring or 3- to 6-membered heterocyclic ring is optionally substituted with 1, 2, 3, or 4 R_{bb} groups, and other variables are as defined herein.

In some embodiments, two R_{b} groups may be attached to different carbon atoms and, together with the carbon to which they are attached, form a C₃₋₆ cycloalkyl ring or a 3- to 6-membered heterocyclic ring, wherein the C₃₋₆ cycloalkyl ring or 3- to 6-membered heterocyclic ring is optionally substituted with 1, 2, 3, or 4 R_{bb} groups, and other variables are as defined herein.

In some embodiments, R_{bb} is independently selected from a halogen, a hydroxyl group, a cyano group, SF₅, -S(=O)₂R, a C₁₋₆ alkyl group, a C₂₋₆ alkenyl group, a C₂₋₆ alkynyl group, a C₁₋₆ heteroalkyl group, a C₂₋₆ heteroalkenyl group, a C₂₋₆ heteroalkynyl group, a phenyl group, a 5- to 9-membered heteroaryl group, a C₃₋₆ cycloalkyl group, and a 3- to 6-membered heterocyclyl group, and other variables are as defined herein.

In some embodiments, R_{bbb} is independently selected from a halogen, a hydroxyl group, a cyano group, SF₅, and a C₁₋₆ alkyl group, and other variables are as defined herein.

In some embodiments, ring B is selected from 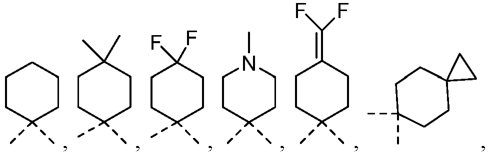 and 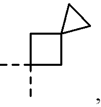 and other variables are as defined herein.

In some embodiments, R_{Y1}, R_{Y2}, and R_{Y4} are each independently selected from hydrogen, a halogen, a hydroxyl group, a cyano group, SF₅, a C₁₋₆ alkyl group, a C₁₋₆ alkoxy group, a C₁₋₆ alkylamino group, and a C₁₋₆ alkylthio group, wherein the C₁₋₆ alkyl group, C₁₋₆ alkoxy group, C₁₋₆ alkylamino group, or C₁₋₆ alkylthio group is optionally substituted with 1, 2, 3, or 4 R_{Y} groups, and other variables are as defined herein.

In some embodiments, R_{Y3} is independently selected from Z-R_{Y3a}, wherein Z is selected from a single bond, -C₀₋₆ alkyl-S-C₀₋₆ alkyl-, -C₀₋₆ alkyl-S(=O)-C₀₋₆ alkyl-, -C₀₋₆ alkyl-S(=O)₂-C₀₋₆ alkyl-, -C₀₋₆ alkyl-NR_{Y3b}-C₀₋₆ alkyl-, -C₀₋₆ alkyl-NR_{Y3b}-S(=O)₂-C₀₋₆ alkyl-, -C₀₋₆ alkyl-S(=O)₂-NR_{Y3b}-C₀₋₆ alkyl-, -C₀₋₆ alkyl-O-C₀₋₆ alkyl-, -C₀₋₆ alkyl-C(=O)-C₀₋₆ alkyl-, -C₀₋₆ alkyl-C(=O)-O-C₀₋₆ alkyl-, -C₀₋₆ alkyl-C(=O)-NR_{Y3b}-C₀₋₆ alkyl-, - C₀₋₆ alkyl-S(=O)(=NH)-, -N=S(=O)-, -(C=O)-, and -C(=N-OH)-, wherein the -C₀₋₆ alkyl-S-C₀₋₆ alkyl-, -C₀₋₆ alkyl-S(=O)-C₀₋₆ alkyl-, -C₀₋₆ alkyl-S(=O)₂-C₀₋₆ alkyl-, -C₀₋₆ alkyl-NR_{Y3b}-C₀₋₆ alkyl-, -C₀₋₆ alkyl-NR_{Y3b}-S(=O)₂-C₀₋₆ alkyl-, -C₀₋₆ alkyl-S(=O)₂-NR_{Y3b}-C₀₋₆ alkyl-, -C₀₋₆ alkyl-O-C₀₋₆ alkyl-, -C₀₋₆ alkyl-C(=O)-C₀₋₆ alkyl-, -C₀₋₆ alkyl-C(=O)-O-C₀₋₆ alkyl-, -C₀₋₆ alkyl-C(=O)-NR_{Y3b}-C₀₋₆ alkyl-, or -C₀₋₆ alkyl-S(=O)(=NH)- is optionally substituted with 1, 2, 3, or 4 R_{Y} groups.
R_{Y3b} is independently selected from hydrogen, a halogen, a hydroxyl group, a cyano group, SF₅, and a C₁₋₆ alkyl group;
R_{Y3a} is independently selected from hydrogen, a halogen, a hydroxyl group, a cyano group, SF₅, a C₁₋₆ alkyl group, a saturated, partially saturated, or unsaturated monocyclic ring composed of 3, 4, 5, 6, or 7 members, or a bicyclic ring composed of 8, 9, 10, 11, or 12 members, comprising 0, 1, 2, or 3 N atoms and 0 or 1 atom selected from O and S, wherein the monocyclic ring or bicyclic ring is optionally substituted with 1, 2, 3, or 4 R' groups;
In some embodiments, Z is selected from a single bond, -NH-S(=O)₂-C₀₋₄ alkyl-, -NH-C₀₋₄ alkyl-, - S(=O)₂-NH-C₀₋₄ alkyl-, and -C₀₋₄ alkyl-S(=O)(=NH)-, and other variables are as defined herein.

In some embodiments, R_{Y3a} is independently selected from hydrogen, a halogen, a hydroxyl group, a cyano group, SF₅, a C₁₋₆ alkyl group, a C₃₋₆ cycloalkyl group, and a 5- to 6-membered heterocyclyl group, wherein the C₁₋₆ alkyl group, C₃₋₆ cycloalkyl group, or 5- to 6-membered heterocyclyl group is optionally substituted with 1, 2, 3, or 4 R' groups, and other variables are as defined herein.

In some embodiments, R' is independently selected from hydrogen, a halogen, a hydroxyl group, a cyano group, SF₅, and a C₁₋₆ alkyl group; alternatively, two R' groups are attached to the same carbon atom to form =C(F)₂ or =O, and other variables are as defined herein.

In some embodiments, R_{Y3} is independently selected from 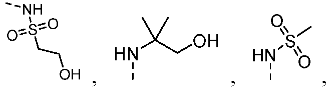 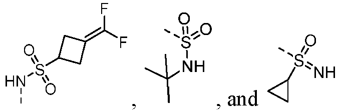 , and other variables are as defined herein.

**In** some embodiments, L₁ is selected from a single bond, a C₁₋₆ alkyl group, a C₂₋₆ alkenyl group, a C₂₋₆ alkynyl group, a C₁₋₆ heteroalkyl group, a C₂₋₆ heteroalkenyl group, and a C₂₋₆ heteroalkynyl group, wherein the C₁₋₆ alkyl group, C₂₋₆ alkenyl group, C₂₋₆ alkynyl group, C₁₋₆ heteroalkyl group, C₂₋₆ heteroalkenyl group, or C₂₋₆ heteroalkynyl group is optionally substituted with 1, 2, 3, or 4 R_{L1} groups, and other variables are as defined herein.

In some embodiments, L₁ is selected from a single bond, -CH₂- and -CH₂CH₂-, wherein the -CH₂- or - CH₂CH₂- is optionally substituted with 1 or 2 R_{L1} groups, and other variables are as defined herein.

In some embodiments, L₂ is selected from a single bond, O, S, N(R_{L}), N(R_{L})C(=O), a C₁₋₆ alkyl group, a C₂₋₆ alkenyl group, a C₂₋₆ alkynyl group, a C₁₋₆ heteroalkyl group, a C₂₋₆ heteroalkenyl group, and a C₂₋₆ heteroalkynyl group, wherein the N(R_{L}), N(R_{L})C(=O), C₁₋₆ alkyl group, C₂₋₆ alkenyl group, C₂₋₆ alkynyl group, C₁₋₆ heteroalkyl group, C₂₋₆ heteroalkenyl group, or C₂₋₆ heteroalkynyl group is optionally substituted with 1, 2, 3, or 4 R_{L2} groups, and other variables are as defined herein.

In some embodiments, L₂ is selected from a single bond, O, S, -CH₂- and -CH₂CH₂-, wherein the -CH₂- or -CH₂CH₂- is optionally substituted with 1 or 2 R_{L1} groups, and other variables are as defined herein.

In some embodiments, the present disclosure provides a compound with the following structure: 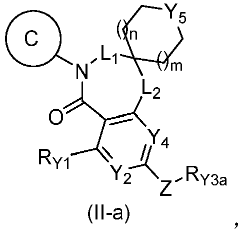
wherein, m is selected from 0, 1, 2, or 3;
n is selected from 0, 1, 2, or 3;
Y₅ is selected from NR_{Y5} and C(R₂)(R₃);
R_{Y5}, R₂, and R₃ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 R_{Y5aa} groups;
alternatively, R₂, R₃, and the carbon to which they are attached form a saturated, partially unsaturated, or unsaturated 5- to 7-membered monocyclic ring, or a saturated, partially unsaturated, or unsaturated 9- to 15-membered bicyclic ring, wherein the monocyclic ring or bicyclic ring is optionally substituted with 1, 2, 3, or 4 R_{Y5aa} groups;
R_{Y5aa} is independently selected from a halogen, a hydroxyl group, a cyano group, SF₅, C₁₋₆ alkyl group, C₂₋₆ alkenyl group, and C₂₋₆ alkynyl group, wherein the C₁₋₆ alkyl group, C₂₋₆ alkenyl group, or C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group, and other variables are as defined herein.

In some embodiments, the present disclosure provides a compound with the following structure: 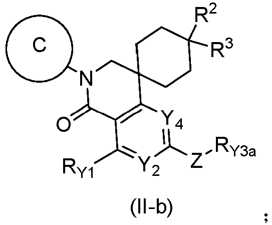
R₂ and R₃ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 R_{Y5aa} groups;
alternatively, R₂, R₃, together with the carbon to which they are attached, form a C₃₋₁₂ cycloalkyl group or a 3- to 12-membered heterocyclic ring, wherein the C₃₋₁₂ cycloalkyl group or 3- to 12-membered heterocyclic ring is optionally substituted with 1, 2, 3, or 4 R_{Y5aa} groups;
R_{Y5aa} is independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, and C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group, and other variables are as defined herein.

In some embodiments, the present disclosure provides a compound with the following structure: 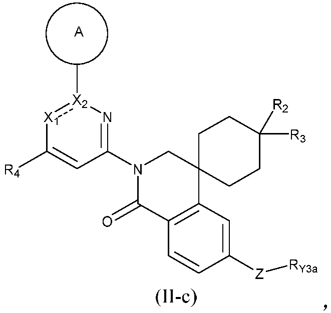
wherein, 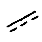 represents a single bond or a double bond;
when 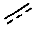 represents a single bond, X₁ is selected from 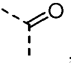 , C(R_{X1})₂, and NR_{X1}, and X₂ is selected from N or CR_{X2};
when 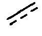 represents a double bond, X₁ is selected from CR_{X1} or N, and X₂ is selected from C;
ring A is selected from a 5- to 12-membered heterocycloalkyl group or a C₃₋₁₂ cycloalkyl group, wherein the 5- to 12-membered heterocycloalkyl group or the C₃₋₁₂ cycloalkyl group is optionally substituted with 1, 2, 3, or 4 R_{A} groups;
R_{X1} and R_{X2} are each independently selected from hydrogen, a halogen, a hydroxyl group, a cyano group, SF₅, a C₁₋₆ alkyl group, a C₂₋₆ alkenyl group, and a C₂₋₆ alkynyl group;
R_{A} is independently selected from hydrogen, a halogen, a hydroxyl group, a cyano group, SF₅, a C₁₋₆ alkyl group, a C₂₋₆ alkenyl group, and a C₂₋₆ alkynyl group;
alternatively, R_{X1} and one of the R_{A} groups, together with the carbon to which they are attached, form a saturated, partially unsaturated, or unsaturated 5- to 9-membered monocyclic ring, wherein the monocyclic ring is optionally substituted with 1, 2, 3, or 4 R_{B} groups;
R_{B} is independently selected from hydrogen, a halogen, a hydroxyl group, a cyano group, SF₅, a C₁₋₆ alkyl group, a C₂₋₆ alkenyl group, and a C₂₋₆ alkynyl group;
R₂, R₃, and R₄ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 R_{Y5aa} groups;
alternatively, R₂, R₃, together with the carbon to which they are attached, form a C₃₋₁₂ cycloalkyl ring or a 5- to 12-membered heterocyclic ring, wherein the C₃₋₁₂ cycloalkyl ring or 5- to 12-membered heterocyclic ring is optionally substituted with 1, 2, 3, or 4 R_{Y5aa} groups;
R_{Y5aa} is independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, and C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group, and other variables are as defined herein.

In some embodiments, the present disclosure provides a compound represented by Formula I, or a pharmaceutically acceptable salt thereof, 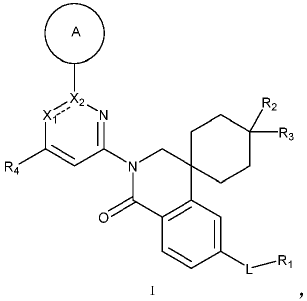
wherein, 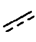 represents a single bond or a double bond;
when 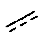 represents a single bond, X₁ is selected from 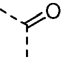 , C(R_{X1})₂, and NR_{X1}, and X₂ is selected from N or CR_{X2};
when 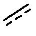 represents a double bond, X₁ is selected from CR_{X1} or N, and X₂ is selected from C;
ring A is selected from a 5- to 6-membered heterocycloalkyl group or a C₅₋₆ cycloalkyl group, wherein the 5- to 6-membered heterocycloalkyl group or the C₅₋₆ cycloalkyl group is optionally substituted with 1, 2, 3, or 4 R_{A} groups;
R_{X1} and R_{X2} are each independently selected from hydrogen, a halogen, a hydroxyl group, a cyano group, SF₅, a C₁₋₆ alkyl group, a C₂₋₆ alkenyl group, and a C₂₋₆ alkynyl group;
R_{A} is independently selected from hydrogen, a halogen, a hydroxyl group, a cyano group, SF₅, a C₁₋₆ alkyl group, a C₂₋₆ alkenyl group, and a C₂₋₆ alkynyl group;
alternatively, R_{X1} and one of the R_{A} groups, together with the carbon to which they are attached, form a saturated, partially unsaturated, or unsaturated 5- to 6-membered monocyclic ring, wherein the monocyclic ring is optionally substituted with 1, 2, 3, or 4 R_{B} groups;
R_{B} is independently selected from hydrogen, a halogen, a hydroxyl group, a cyano group, SF₅, a C₁₋₆ alkyl group, a C₂₋₆ alkenyl group, and a C₂₋₆ alkynyl group;
R₂, R₃, and R₄ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 R_{Y5aa} groups;
alternatively, R₂, R₃, together with the carbon to which they are attached, form a C₃₋₆ cycloalkyl ring or a 3- to 6-membered heterocyclic ring, wherein the C₃₋₆ cycloalkyl ring or 3- to 6-membered heterocyclic ring is optionally substituted with 1, 2, 3, or 4 R_{Y5aa} groups;
R_{Y5aa} is independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group;
L is selected from a single bond, -C₀₋₄ alkyl-S-C₀₋₄ alkyl-, -C₀₋₄ alkyl-S(=O)-C₀₋₄ alkyl-, -C₀₋₄ alkylS(=O)₂-C₀₋₄ alkyl-, -C₀₋₄ alkyl-NR_{Y3b}-C₀₋₄ alkyl-, -C₀₋₄ alkyl-NR_{Y3b}-S(=O)₂-C₀₋₄ alkyl-, -C₀₋₄ alkyl-S(=O)₂-NR_{Y3b}-₀₋₄ alkyl-, -C₀₋₄ alkyl-O-C₀₋₄ alkyl-, -C₀₋₄ alkyl-C(=O)-C₀₋₄ alkyl-, -C₀₋₄ alkyl-C(=O)-O-C₀₋₄ alkyl-, -C₀₋₄ alkyl-C(=O)-NR_{Y3b}-C₀₋₄ alkyl-, -C₀₋₄ alkyl-S(=O)(=NH)-, -N=S(=O)-, -(C=O)-, or -C(=N-OH)-;
R_{Y3b} is independently selected from hydrogen, a halogen, a hydroxyl group, a cyano group, SF₅, and a C₁₋₆ alkyl group;
R₁ is independently selected from hydrogen, a halogen, a hydroxyl group, a cyano group, SF₅, a C₁₋₆ alkyl group, a C₃₋₆ cycloalkyl group, and a 5-6 membered heterocyclyl group, wherein the C₁₋₆ alkyl group, C₃₋₆ cycloalkyl group, or 5-6 membered heterocyclyl group is optionally substituted with 1, 2, 3, or 4 R' groups;
R' is independently selected from hydrogen, a halogen, a hydroxyl group, a cyano group, SF₅, and a C₁₋₆ alkyl group;
alternatively, two R' groups are attached to the same carbon atom to form =C(F)₂ or =O, and other variables are as defined herein.

In some embodiments, the present disclosure provides a compound represented by Formula I, or a pharmaceutically acceptable salt thereof, 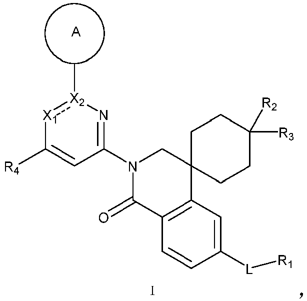 wherein: 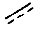
represents a single bond or a double bond;
when 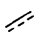 represents a single bond, X₁ is selected from 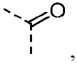 and X₂ is selected from N;
when 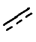 represents a double bond, X₁ is selected from CH or N, and X₂ is selected from C;
ring A is selected from a 4- to 6-membered heterocycloalkyl group or a C₃₋₆ cycloalkyl group, wherein the 4- to 6-membered heterocycloalkyl group or the C₃₋₆ cycloalkyl group is optionally substituted with 1 or 2 groups each independently selected from a halogen or a C₁₋₃ alkyl group;
-L-R₁ is selected from 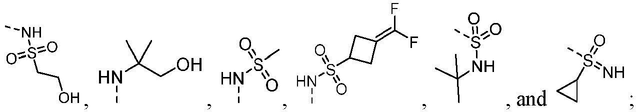
R₂, R₃, and R₄ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, and C₂₋₆ alkynyl group is optionally substituted with 1, 2, or 3 R_{Y5aa} groups;
alternatively, R₂, R₃, together with the carbon to which they are attached, form a C₃₋₆ cycloalkyl ring or a 5- to 6-membered heterocyclic ring, wherein the C₃₋₆ cycloalkyl ring or 5- to 6-membered heterocyclic ring is optionally substituted with 1, 2, 3, or 4 R_{Y5aa} groups;
R_{Y5aa} is independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, and C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group, and other variables are as defined herein.

In some embodiments, the present disclosure provides a compound represented by Formula I, or a pharmaceutically acceptable salt thereof, 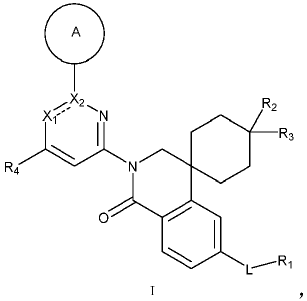 wherein: 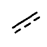
represents a single bond or a double bond;
when 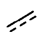 represents a single bond, X₁ is selected from 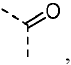 and X₂ is selected from N;
when 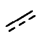 represents a double bond, X₁ is selected from CH or N, and X₂ is selected from C;
ring A is selected from a 6-membered heterocycloalkyl group or a cyclobutyl group, wherein the 6-membered heterocycloalkyl group or the cyclobutyl group is optionally substituted with 1 or 2 groups each independently selected from a halogen or a C₁₋₃ alkyl group;
L is selected from NH;
R₁ is selected from -S(=O)₂-C₁₋₃ alkyl-OH;
R₂ and R₃ are each independently selected from H or a C₁₋₃ alkyl group;
alternatively, R₂, R₃, and the carbon to which they are attached together form a cyclopropyl ring;
R₄ is selected from H or a C₁₋₃ alkyl group, and other variables are as defined herein.

In some embodiments, ring A is selected from a morpholinyl group, a piperidinyl group, or a cyclobutyl group, wherein the morpholinyl group, piperidinyl group, or cyclobutyl group is optionally substituted with 1 or 2 groups independently selected from F or a C₁₋₃ alkyl group, and other variables are as defined herein.

In some embodiments, ring A is selected from 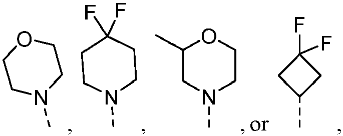 and other variables are as defined herein.

In some embodiments, R₄ is selected from H or a methyl group, and other variables are as defined herein.

In some embodiments, R₂ and R₃ are each independently selected from H or a methyl group, and other variables are as defined herein.

In some embodiments, R₁ is selected from 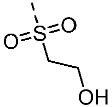 , and other variables are as defined herein.

In some embodiments, the present disclosure provides a compound with the following structure:

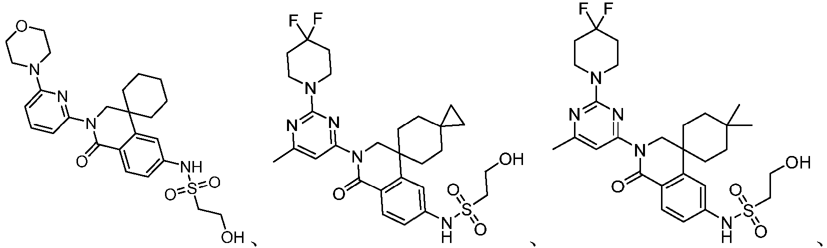

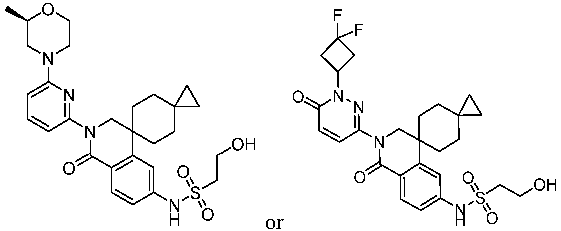

In some embodiments, the present disclosure provides a compound listed in Table a:

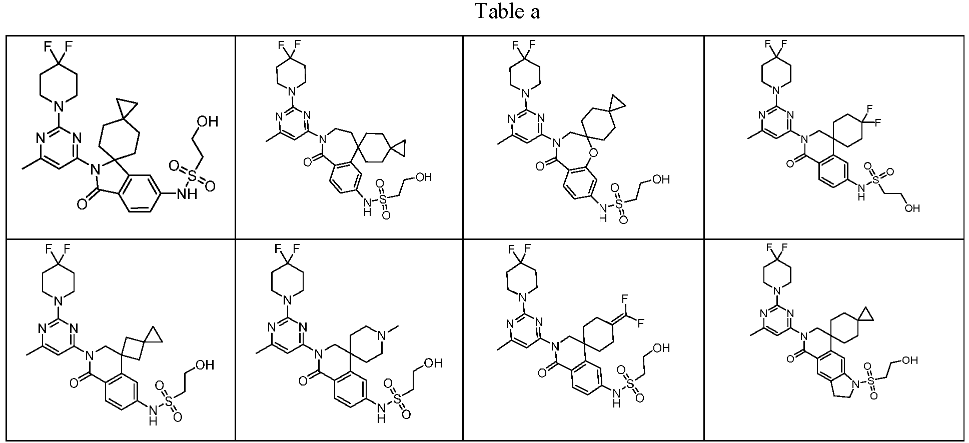

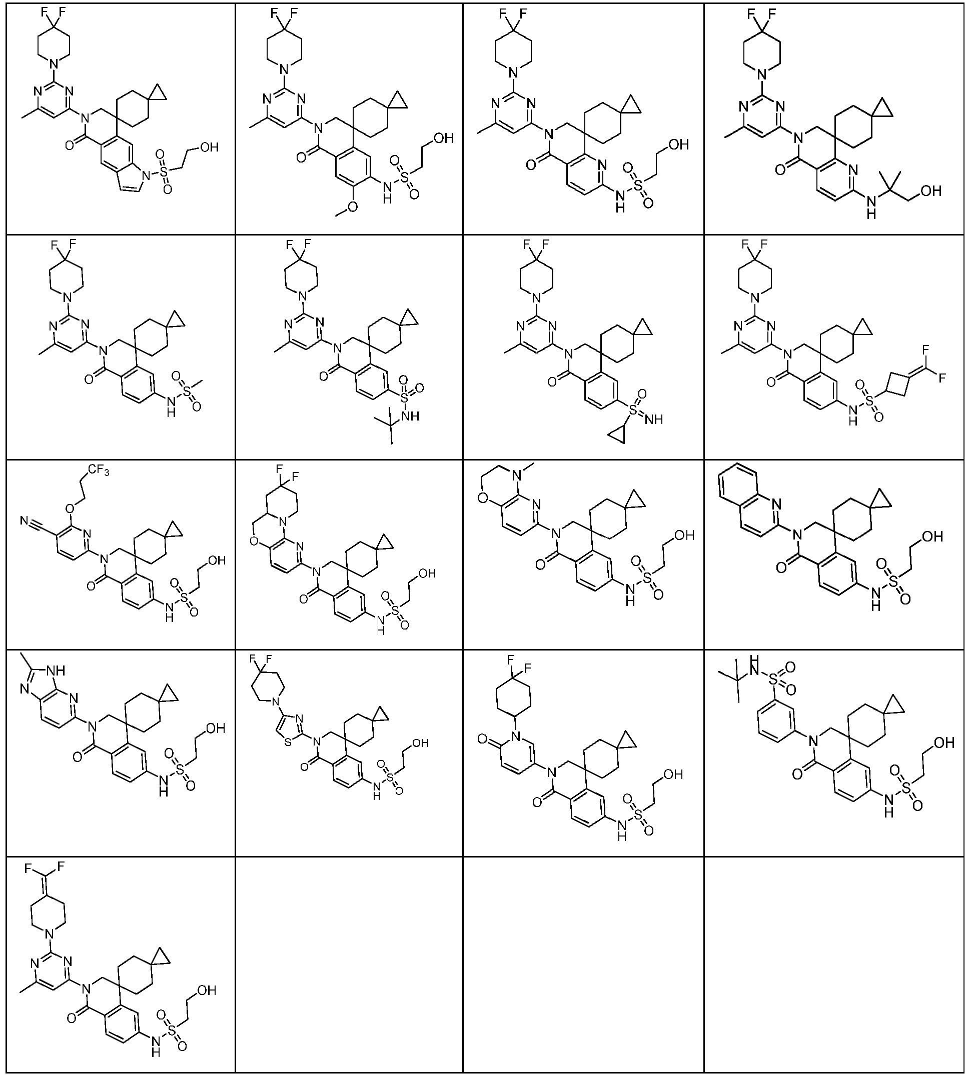

In some embodiments, compounds having the structure of the present disclosure, including hydrogen being replaced with deuterium or tritium, or carbon being replaced with ¹³C- or ¹⁴C-enriched carbon, are within the scope of the present disclosure. For example, in some embodiments, H is deuterium in the substituents of ring C, ring B, L₁, L₂, Y₁, Y₂, Y₃, Y₄, R₂, R₃, L, or R₁.

The present disclosure also provides a pharmaceutical composition comprising a compound of any of the aforementioned formulas or a pharmaceutically acceptable salt thereof, and a pharmaceutically acceptable carrier or excipient.

The present disclosure further provides the use of a substance X in the preparation of a KIF18A inhibitor, wherein the substance X is a compound of any of the aforementioned formulas or a pharmaceutically acceptable salt thereof, or the foregoing pharmaceutical composition.

The present disclosure further provides the use of a substance X in the preparation of a medicament, wherein the substance X is a compound of any of the aforementioned formulas or a pharmaceutically acceptable salt thereof, or the foregoing pharmaceutical composition, and wherein the medicament is for the treatment of cancer.

The positive and progressive effects of the present disclosure are as follows: The spirocyclic derivatives of the present disclosure are structurally novel KIF18A inhibitors.

### Specific Embodiments

Some embodiments are to be described in detail next, with the examples be illustrated in the Specific Embodiments herein. While specific embodiments are described, it should be understood that they are not intended to limit the present disclosure thereto. Rather, the present disclosure intends to cover all alternatives, modifications, and equivalent solutions that may be included within the scope of the present disclosure as defined in the Claims. Those skilled in the art will recognize many methods and materials similar or equivalent to those described herein, which may be used in the practice of the present disclosure. The present disclosure is in no way limited to the methods and materials described. In the event of any differences or inconsistencies between the present disclosure and any incorporated references or similar materials, including but not limited to definitions of terms, terminology usage, and described techniques, the present disclosure shall prevail.

It may be understood that some features of the present disclosure, described for clarity in the context of individual embodiments, may also be provided in combination within a single embodiment. Conversely, various features of the present disclosure described in the context of a single embodiment may also be provided individually or in any suitable sub-combination for the sake of brevity.

### Definitions

Terms used in the present disclosure but not explicitly defined shall have their ordinary meanings, and the meaning of such terms shall be independent each time they appear. However, unless otherwise specified, the following definitions apply to entire Specification and Claims.

As used herein, the terms "comprise" and "include" are intended to specify the presence of the stated features, entirety, components, or steps, but do not exclude the presence or addition of one or more other features, entirety, components, steps, or combinations thereof.

The following provides a more detailed description of the definitions of specific functional groups and chemical terms. For the purposes of the present disclosure, chemical elements are identified according to the Periodic Table of Elements, CAS Handbook of Chemistry and Physics, 75th Edition, inside cover. Specific functional groups are generally defined as described therein. Furthermore, general principles of organic chemistry as well as descriptions of specific functional groups and reactivities are set forth in Organic Chemistry, Thomas Sorrell, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; Carruthers, Some Modern Methods of Organic Synthesis, 3rd Edition, Cambridge University Press, Cambridge, 1987. Unless explicitly stated otherwise, all ranges cited herein are included.

When a range of values is provided, it is intended to encompass every individual value and sub-range within that range. For example, "C₁₆" is intended to cover C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆. Other ranges appearing herein are similar.

When any variable appears more than once in any constituent, in the general formulas describing the compounds of the present disclosure, or in any other formula, its definition at each occurrence is independent of its definition at every other occurrence. Furthermore, combinations of substituents and/or variables are permitted only when such combinations result in stable compounds.

As used herein, the term "substituted" or "substituted with" refers to the replacement of one or more hydrogen atoms on a specified atom with substituent(s), which may include isotopes of deuterium and hydrogen, provided that the valency of the specified atom is maintained and the substituted compound is stable. The term "optionally substituted" or "optionally substituted with" means that substitution may or may not occur. Unless otherwise specified, both the type and number of substituents may be arbitrary when being chemically feasible. As used herein, the term "substituent" has its ordinary meaning as understood in the art and refers to a chemical moiety that is covalently attached to, or, where appropriate, fused to a parent moiety. It may be understood that substitution on a given atom is subject to the limitations of valency. It should be understood that substituents may be further substituted.

As used herein, the term "optional" or "optionally" refers to that the subsequently described event or circumstance may or may not occur, and that the description includes instances where the event or circumstance occurs and instances where it does not.

As used herein, when a group is depicted with a dashed line " 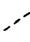 " on a valence bond, for example in 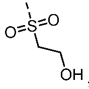 , the dashed line indicates the point of attachment of that group to the remainder of the molecule. As used herein, when a dashed line " 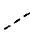 " appears on a single bond, for example in " 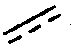 ", the dashed line represents either the presence or absence of a bond. It indicates that the bond may be a single bond " 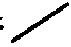 " or a double bond " 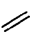 ".

As used herein, the term "alkyl group" refers to a straight-chain or branched saturated hydrocarbon group. The term "Cᵢ₋ⱼ alkyl group" refers to an alkyl group having from i to j carbon atoms, and similar expressions used hereafter may be interpreted in a similar manner. In some embodiments, the alkyl group may comprise 1 to 12 carbon atoms. In some embodiments, the alkyl group may comprise 1 to 6 carbon atoms, for example, 1 to 5 carbon atoms, 1 to 4 carbon atoms, 1 to 3 carbon atoms, or 1 to 2 carbon atoms; the alkyl group may be monovalent (e.g., methyl), divalent (e.g., methylene), or polyvalent (e.g., trimethylene). Non-limiting examples of alkyl groups include methyl, ethyl, n-propyl, isopropyl, n-butyl, sec-butyl, isobutyl, tert-butyl, neopentyl, and the like.

As used herein, the term "alkenyl group" refers to a straight-chain or branched hydrocarbon group comprising at least one carbon-carbon double bond, including groups with "cis" and "trans" orientations, or optionally "E" and "Z" configurations. In some embodiments, the alkenyl group may comprise 2 to 12 carbon atoms. In some embodiments, the alkenyl group may comprise 2 to 6 carbon atoms, for example, 2 to 5 carbon atoms, 2 to 4 carbon atoms, or 2 to 3 carbon atoms. Non-limiting examples of alkenyl groups include vinyl (ethenylene), propenyl, butenyl, pentenyl, 1-methyl-2-buten-1-yl, 5-hexenyl, and the like.

As used herein, the term "alkynyl group" refers to a straight-chain or branched hydrocarbon group having at least one carbon-carbon triple bond. In some embodiments, the alkynyl group may comprise 2 to 12 carbon atoms. In some embodiments, the alkynyl group may comprise 2 to 6 carbon atoms, 2 to 5 carbon atoms, 2 to 4 carbon atoms, or 2 to 3 carbon atoms. Non-limiting examples of alkynyl groups include ethynyl, 1-propynyl, 2-propynyl, and the like.

As used herein, the term "heteroalkyl group" refers to a straight-chain or branched saturated hydrocarbon group, wherein at least one carbon atom is replaced with a heteroatom or heteroatomic group selected from O, NH, S, C(=O), C(=O)O, S(=O), S(=O)₂, S(=O)(=NH), and N. In some embodiments, the heteroalkyl group may comprise 1 to 12 carbon atoms. In some embodiments, the heteroalkyl group may comprise 1 to 6 carbon atoms, for example, 1 to 5 carbon atoms, 1 to 4 carbon atoms, 1 to 3 carbon atoms, or 1 to 2 carbon atoms. Non-limiting examples of heteroalkyl groups include alkoxy, alkylamino, alkylthio, and the like. The heteroalkyl group may be substituted or unsubstituted.

As used herein, the term "heteroalkenyl group" refers to a straight-chain or branched hydrocarbon group having at least one carbon-carbon double bond, wherein at least one carbon atom is substituted with a heteroatom or heteroatomic group selected from O, NH, S, C(=O), C(=O)O, S(=O), S(=O)₂, S(=O)(=NH), and N. In some embodiments, the heteroalkenyl group may comprise 2 to 12 carbon atoms. In some embodiments, the heteroalkenyl group may comprise 2 to 6 carbon atoms, for example, 2 to 5 carbon atoms, 2 to 4 carbon atoms, or 2 to 3 carbon atoms. A heteroalkenyl group may be substituted or unsubstituted.

As used herein, the term "heteroalkenyl group" refers to a straight-chain or branched hydrocarbon group having at least one carbon-carbon triple bond, wherein at least one carbon atom is substituted with a heteroatom or heteroatomic group selected from O, NH, S, C(=O), C(=O)O, S(=O), S(=O)₂, S(=O)(=NH), and N. In some embodiments, the heteroalkenyl group may comprise 2 to 12 carbon atoms. In some embodiments, the heteroalkenyl group may comprise 2 to 6 carbon atoms, for example, 2 to 5 carbon atoms, 2 to 4 carbon atoms, or 2 to 3 carbon atoms. A heteroalkynyl group may be substituted or unsubstituted.

As used herein, the term "alkoxy group" refers to a group-O-alkyl, wherein the alkyl group has the meaning as defined herein.

As used herein, the term "aryl group" refers to a monocyclic, bicyclic, or polycyclic carbocyclic system having at least one aromatic ring. In some embodiments, the aryl group may comprise 6 to 12 members. In some embodiments, the aryl group may comprise six ring carbon atoms. All atoms within a carbocyclic aryl group are carbon atoms. Non-limiting examples of aryl groups include phenyl, naphthyl, 1,2-dihydronaphthyl, 1,2,3,4-tetrahydronaphthyl, fluorenyl, indanyl, indenyl, and the like.

As used herein, the term "heteroaryl group" refers to a monocyclic system, or a fused or bridged bicyclic or polycyclic system, wherein the ring system comprises one, two, three, four, or more heteroatoms independently selected from the group consisting of nitrogen, oxygen, and sulfur; and at least one of the rings is an aromatic ring. In some embodiments, the heteroaryl group may comprise 5 to 12 members. In some embodiments, the heteroaryl group may comprise one, two, or three heteroatoms. In some embodiments, the heteroaryl group may comprise one or two heteroatoms. Non-limiting examples of heteroaryl groups include benzimidazolyl, benzofuranyl, benzothiazolyl, benzothiophenyl, benzoxazolyl, furanyl, imidazolyl, indolyl, isoindolyl, isoquinolinyl, isothiazolyl, isoxazolyl, oxadiazolyl, oxazolyl, purinyl, pyrrolyl, pyridinyl, pyrazinyl, pyrimidinyl, quinolinyl, thiadiazolyl, thiazolyl, thienyl, triazolyl, tetrazolyl, dihydroindolyl, tetrahydroquinolinyl, tetrahydroisoquinolinyl, and the like. A heteroaryl group comprises at least one ring having at least one of the aforementioned heteroatoms and at least one aromatic ring. For example, a ring having at least one heteroatom may be fused to one, two, or three carbocyclic rings, such as an aryl ring, cyclohexane ring, cyclohexene ring, cyclopentane ring, cyclopentene ring, or another monocyclic heterocyclic ring. Non-limiting examples of fused heteroaryl groups include 2,3-dihydrobenzofuran, 2,3-dihydroindole, 2,3-dihydrobenzothiophene, and the like.

Unless otherwise specified, the number of atoms on a ring is typically defined as the number of members of the ring, for example, a "3- to 6-membered ring" refers to a "ring" composed of 3 to 6 atoms arranged in a cyclic structure.

As used herein, the term "cycloalkyl group" refers to a non-aromatic saturated or partially unsaturated monocyclic or polycyclic ring system, wherein all ring atoms are carbon. In some embodiments, the cycloalkyl group may comprise 3 to 12 ring carbon atoms (i.e., C₃₋₁₂ cycloalkyl group). In some embodiments, the cycloalkyl group may comprise 3 to 11, 3 to 10, 3 to 9, 3 to 8, 3 to 7, 3 to 6, 4 to 10, 4 to 9, 4 to 8, 4 to 7, 4 to 6, 4 to 5, 5 to 10, 5 to 9, 5 to 8, 5 to 7, or 5 to 6 ring carbon atoms, and the like. In particular, the cycloalkyl group may be monocyclic or bicyclic. Optionally, the bicyclic cycloalkyl group may comprise fused, spirocyclic, or bridged cycloalkyl structures.

As used herein, the term "monocyclic" refers to a saturated or unsaturated ring structure arranged in a single closed ring form. In some embodiments, the monocyclic ring may comprise 3 to 10 ring atoms, for example, 3, 4, 5, 6, 7, 8, 9, or 10 ring atoms. In some embodiments, the ring atoms may be carbon atoms or heteroatoms. In some embodiments, the monocyclic ring structure may further be substituted with substituent(s).

As used herein, the term "bicyclic" refers to a saturated or unsaturated ring system arranged as two connected closed rings. In some embodiments, the bicyclic ring may comprise 4 to 14 ring atoms, for example, 4, 5, 6, 7, 8, 9, 10, 11, 12, 13, or 14 ring atoms. Optionally, the bicyclic ring may be a fused bicyclic ring, a spirocyclic bicyclic ring, or a bridged bicyclic ring. In some embodiments, the ring atoms may be carbon atoms or heteroatoms. In some embodiments, the bicyclic ring structure may further be substituted with substituent(s).

As used herein, the term "fused ring" refers to a polycyclic structure comprising two or more monocyclic rings, wherein each pair of adjacent monocyclic rings shares two ring atoms. The monocyclic rings included in the fused ring may be saturated or unsaturated, aromatic or non-aromatic. In some embodiments, the fused ring may comprise 4 to 18 ring atoms. In some embodiments, the fused ring may comprise 8 to 16 ring atoms, for example, 8, 9, 10, 11, 12, 13, 14, 15, or 16 ring atoms. In some embodiments, the ring atoms may be carbon atoms or heteroatoms. In some embodiments, the fused ring structure may further be substituted with substituent(s).

As used herein, the term "spirocyclic ring" refers to a polycyclic structure comprising two or more monocyclic rings, wherein each pair of adjacent monocyclic rings shares one ring atom. The monocyclic rings included in the spirocyclic ring may be saturated or unsaturated, aromatic or non-aromatic. In some embodiments, the spirocyclic ring may comprise 5 to 18 ring atoms. In some embodiments, the fused [sic: spirocyclic] ring may comprise 7 to 16 ring atoms, for example, 7, 8, 9, 10, 11, 12, 13, 14, 15, or 16 ring atoms. In some embodiments, the ring atoms may be carbon atoms or heteroatoms. In some embodiments, the fused ring structure may further be substituted with substituent(s).

As used herein, the term "heterocyclyl group" or "heterocyclic ring" refers to a group formed by a cycloalkyl structure as defined above, wherein at least one ring atom is a heteroatom selected from N, O, and S. In some embodiments, the heterocyclyl group may comprise 3 to 12 ring atoms (i.e., a 3- to 12-membered heterocyclyl group). In some embodiments, the heterocyclyl group may comprise 3 to 11, 3 to 10, 3 to 9, 3 to 8, 3 to 7, 3 to 6, 4 to 10, 4 to 9, 4 to 8, 4 to 7, 4 to 6, 4 to 5, 5 to 10, 5 to 9, 5 to 8, 5 to 7, or 5 to 6 ring atoms, and the like. Specifically, the heterocyclyl group may be a saturated or partially unsaturated monocyclic or bicyclic structure. Optionally, the bicyclic heterocyclyl group may comprise fused, spirocyclic, or bridged heterocyclic structures. Non-limiting examples of heterocyclyl groups include epoxyethyl, pyrrolidinyl, piperidinyl, tetrahydropyranyl, piperazinyl, pyrrolidinyl, and morpholinyl. A heterocyclyl group may also be described by using carbon number. For example, a C₃₋₆ heterocyclyl group refers to a heterocyclic group comprising three to six ring carbon atoms and may further comprise at least one heteroatom, such as 1, 2, or 3 heteroatoms, as ring atoms. In some embodiments, the heterocyclyl group or heterocyclic ring comprises 1 or 2 heteroatoms as ring atoms. In some embodiments, the heterocyclyl group may be monocyclic or bicyclic, such as a fused bicyclic or spirobicyclic ring. As used in the context of the present disclosure, the terms "heterocyclyl group" and "heterocyclic ring" may be used interchangeably.

As used herein, the term "halogen" refers to fluorine (F), chlorine (Cl), bromine (Br), or iodine (I). In some embodiments, non-limiting examples of halogen include fluorine, chlorine, and bromine, and more particularly fluorine and chlorine.

As used herein, the term "heteroatom" refers to nitrogen (N), oxygen (O), and sulfur (S), and may include any oxidized forms of nitrogen and sulfur (i.e., NO and S(O)ₚ, where p is 1 or 2), as well as any quaternized forms of basic nitrogen, unless otherwise specified.

When one of the variables is selected from a single bond, it indicates that the two groups to which it is attached are directly linked. For example, when L₁ in 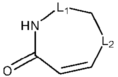 is selected from a single bond, the structure thereof is

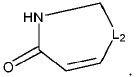

The technical and scientific terms not specifically defined as used herein have the meanings commonly understood by those skilled in the art to which the present disclosure pertains.

The compounds provided herein are described with reference to general formulas and specific compounds. Furthermore, the compounds of the present disclosure may exist in various forms or derivatives, all of which fall within the scope of the present disclosure. These include, for example, pharmaceutically acceptable salts, tautomers, stereoisomers, racemic mixtures, positional isomers, prodrugs, solvates, different crystal forms or polymorphs, and active metabolites.

As used herein, unless otherwise specified, the term "pharmaceutically acceptable salt" includes salts that retain the biological efficacy of the free acid/base form of a given compound and are not undesirable biologically or otherwise. A pharmaceutically acceptable salt may include salt formed with inorganic bases or acids as well as organic bases or acids. Where the compounds of the present disclosure comprise one or more acidic or basic groups, the present disclosure also encompasses their corresponding pharmaceutically acceptable salts. Therefore, the compounds of the present disclosure comprising an acidic group (such as a carboxyl group) may exist in salt form and may be used according to the present disclosure, for example, as an alkali metal salt, alkaline earth metal salt, aluminum salt, or ammonium salt. Further non-limiting examples of such salts include lithium salts, sodium salts, potassium salts, calcium salts, magnesium salts, barium salts, or salts with ammonia or organic amines (such as ethylamine, ethanolamine, diethanolamine, triethanolamine, piperidine, N-methyl-L-glutamine, or amino acids). For example, these salts are readily obtained by letting a compound comprising an acidic group react with a suitable base (such as lithium hydroxide, sodium hydroxide, sodium propoxide, potassium hydroxide, potassium ethoxide, magnesium hydroxide, calcium hydroxide, or barium hydroxide). Other basic salts of the compounds of the present disclosure include, but are not limited to, copper(I), copper(II), iron(II), iron(III), manganese(II), and zinc salts. The compounds of the present disclosure comprising one or more basic groups, such as a protonatable group, may exist in salt form and may be used according to the present disclosure in the form of an addition salt thereof with an inorganic or organic acid. Examples of suitable acids include hydrochloric acid, hydrobromic acid, hydroiodic acid, phosphoric acid, sulfuric acid, nitric acid, methanesulfonic acid, p-toluenesulfonic acid, naphthalenedisulfonic acid, sulfoacetic acid, trifluoroacetic acid, oxalic acid, acetic acid, tartaric acid, lactic acid, salicylic acid, benzoic acid, carbonic acid, formic acid, propionic acid, trimethylacetic acid, diethylacetic acid, malonic acid, succinic acid, heptanedioic acid, fumaric acid, maleic acid, malic acid, pamoic acid, mandelic acid, amidosulfonic acid, phenylpropanoic acid, glucuronic acid, ascorbic acid, isonicotinic acid, citric acid, adipic acid, taurocholic acid, glutaric acid, stearic acid, glutamic acid, or aspartic acid, as well as other acids known to those skilled in the art. The formed salts particularly include hydrochloride salts, chlorides, hydrobromide salts, bromides, iodides, sulfates, phosphates, methanesulfonates (mesylates), p-toluenesulfonates, carbonates, bicarbonates, formates, acetates, sulfoacetates, trifluoromethanesulfonates, oxalates, malonates, maleates, succinates, tartrates, malates, pamoates, mandelates, fumarates, lactates, citrates, glutarates, stearates, aspartates, or glutamates. In addition, the stoichiometry of the salts formed from the compounds of the present disclosure may be an integer or non-integer multiple of 1.

The compounds of the present disclosure comprising a basic nitrogen-containing group may be quaternized using a reagent such as a C₁₋₄ haloalkane, for example, a methyl-, ethyl-, isopropyl-, and tert-butyl-chloride, -bromide, and -iodide; a di-C₁₋₄ alkyl sulfate, such as dimethyl sulfate, diethyl sulfate, and dipentyl sulfate; a C₁₀₋₁₈ alkyl halide, such as chloride, bromide, and iodide of decyl, dodecyl, lauryl, myristyl, and stearyl; and an aryl-C₁₋₄ alkyl halide, such as benzyl chloride and phenethyl bromide.

If the compounds of the present disclosure comprise both acidic and basic groups in the molecule, the present disclosure further encompasses inner salts or betaines (zwitterions) in addition to the aforementioned salt forms. The corresponding salts may be obtained by using conventional methods known to those skilled in the art, for example, by letting them contact with an organic or inorganic acid or base in a solvent or dispersant, or by letting them exchange with the anion or cation of other salts. The present disclosure further includes all salts of the compounds that, due to low physiological compatibility, are not directly suitable for pharmaceutical use but may be used, for example, as intermediates in chemical reactions or in the preparation of pharmaceutically acceptable salts. For a review of more suitable salts, see Stahl and Wermuth, Handbook of Pharmaceutical Salts: Properties, Selection, and Use* (Wiley-VCH, 2002).

The compounds of the present disclosure and the pharmaceutically acceptable salts thereof may exist in both non-solvated and solvated forms. As used herein, the term "solvate" refers to a molecular complex comprising the compound of the present disclosure or a pharmaceutically acceptable salt thereof and one or more molecules of a pharmaceutically acceptable solvent. For example, the term "hydrate" is used when the solvent is water.

The compounds of the present disclosure may have one or more chiral (asymmetric) centers. All stereoisomeric forms of the compounds of the present disclosure are encompassed by the present disclosure. The asymmetric center(s) present in the compounds of the present disclosure may independently have the *(R)* or (S) configuration. When a bond to a chiral carbon is depicted as a straight line in a structural formula of the present disclosure, or when a compound name is presented without specifying (R) or (S) configuration at the chiral center(s), it should be understood that both the (R) and (S) configurations at each such chiral center, and thus each enantiomer, diastereomer, and any mixtures thereof, are included within the scope of the formula or name. The production of specific stereoisomers or mixtures thereof may be identified in embodiments describing the preparation of such stereoisomers or mixtures; however, this in no way limits the inclusion of all stereoisomers and mixtures thereof within the scope of the present disclosure.

The present disclosure encompasses all possible enantiomers and diastereomers, as well as mixtures of two or more stereoisomers, such as mixtures of enantiomers and/or diastereomers in any ratio. Therefore, the enantiomers of the subject matter of the present disclosure are included as pure enantiomeric forms (as levorotatory and dextrorotatory enantiomers), racemic mixtures, and mixtures of the two enantiomers in all possible ratios. In the case of cis/trans isomerism, the present disclosure includes both the cis and trans forms as well as mixtures of these forms in all ratios. If needed, individual stereoisomers may be prepared by separating mixtures using conventional methods (e.g., by chromatography or crystallization, by using stereochemically pure starting materials, or by stereoselective synthesis). Optionally, derivatization may be performed prior to the separation of stereoisomers. Separation of stereoisomeric mixtures may occur at an intermediate stage during the synthesis of the compounds of the present disclosure, or may be carried out on the final racemic product. The absolute stereochemistry may be determined, if needed, by X-ray crystallography of a crystalline product or crystalline intermediate, optionally after derivatization with a reagent comprising a stereocenter of known configuration. Optionally, absolute stereochemistry may be determined by vibrational circular dichroism (VCD) spectroscopy.

Unless otherwise specified, the structures described herein are also intended to include compounds that differ only in the presence of one or more isotopically enriched atoms. In other words, compounds in which one or more atoms are replaced with atoms having the same atomic number but a different atomic mass or mass number than the predominant naturally occurring form. Such compounds are referred to as "isotopically enriched variants". The present disclosure is intended to include all pharmaceutically acceptable isotopically enriched variants of the compounds hereof. Examples of isotopes suitable for inclusion in the compounds of the present disclosure include, but are not limited to, isotopes of hydrogen such as ²H (i.e., D) and ³H; carbon such as ¹¹C, ¹³C, and ¹⁴C; chlorine such as ³⁶Cl; fluorine such as ¹⁸F; iodine such as ¹²³I and ¹²⁵I; nitrogen such as ¹³N and ¹⁵N; oxygen such as ¹⁵O, ¹⁷O, and ¹⁸O; phosphorus such as ³²P; and sulfur such as ³⁵S. Certain isotopically enriched variants of the compounds of the present disclosure, such as those incorporating radioactive isotopes, may be useful in drug and/or substrate tissue distribution studies. In particular, a compound having a depicted structure that differs only by substitution with a heavier isotope (e.g., replacement of hydrogen with deuterium (²H or D)) may offer certain therapeutic advantages, such as increased metabolic stability, extended in vivo half-life, or reduced dosage requirements, and thus may be useful in particular circumstances. Isotopically enriched variants of the compounds of the present disclosure may generally be prepared by conventional techniques known to those skilled in the art or by methods analogous to those described in the accompanying embodiments, using appropriate isotopically labeled reagents in place of the non-labeled reagents previously used.

Pharmaceutically acceptable solvates according to the present disclosure may include those in which the crystallization solvent is isotopically substituted, such as D₂O, d₆-acetone, or d₆-DMSO.

One embodiment of the present disclosure is the administration of the compounds of the present disclosure in the form of a prodrug. Therefore, certain derivatives of the compounds of the present disclosure may have little or no pharmacological activity but are converted into compounds of the present disclosure having the desired activity when administered in vivo or to the body, for example, by hydrolytic cleavage, particularly hydrolysis facilitated by an esterase or a peptidase. Such derivatives are referred to as "prodrugs". More information regarding the use of prodrugs can be found, for example, in T. Higuchi and W. Stella, Prodrugs as Novel Delivery Systems, Vol. 14, ACS Symposium Series, and E. B. Roche (Ed.), Bioreversible Carriers in Drug Design, Pergamon Press, 1987, American Pharmaceutical Association. Further references include Nature Reviews/Drug Discovery, 2008, 7, 355, and Current Opinion in Drug Discovery and Development, 2007, 10, 550.

The prodrugs according to the present disclosure may be prepared, for example, by replacing a suitable functional group present in the compounds of the present disclosure with certain moieties known to those skilled in the art, such as the "promoieties" described in H. Bundgaard, Design of Prodrugs, Elsevier, 1985, and Y. M. Choi-Sledeski and C. G. Wermuth, Designing Prodrugs and Bioprecursors, Practice of Medicinal Chemistry, 4th edition, Chapter 28, pp. 657-696, Elsevier, 2015. Therefore, prodrugs of the present disclosure may include, but are not limited to (a) an ester or amide derivative of a carboxylic acid in the compounds of the present disclosure, if any; (b) an amide, imine, carbamate, or amine derivative of an amino group in the compounds of the present disclosure ;(c) an oxime or imine derivative of a carbonyl group in the compounds of the present disclosure, if any; or (d) a methyl, primary alcohol, or aldehyde group in the compounds of the present disclosure capable of being metabolically oxidized to a carboxylic acid, if any.

The compounds of the present disclosure include the compounds as well as their prodrugs. The present disclosure encompasses such compounds and the pharmaceutically acceptable salts thereof, as well as pharmaceutically acceptable solvates of the compounds and salts.

### Use and Administration

The compounds of the present disclosure-or the pharmaceutically acceptable salts thereof, including mixtures in all ratios-may be used as pharmaceuticals. This series of compounds acts as inhibitors of the KIF18A protein, having excellent enzymatic inhibition activity against the KIF18A protein.

The compounds of the present disclosure as KIF18A protein inhibitors are particularly suitable for treating hyperproliferative diseases such as cancer, including but not limited to colon cancer, breast cancer, lung cancer, pancreatic cancer, prostate cancer, bladder cancer, head cancer, neck cancer, cervical cancer, and ovarian cancer.

As used herein, the term "hyperproliferative disease" refers to a disease characterized by undesired or uncontrolled cell proliferation in a subject. In some embodiments, the hyperproliferative disease is cancer.

The compounds of the present disclosure may be administered in an amount effective to treat the diseases or conditions described herein. The compounds of the present disclosure may be administered as the compounds themselves or, optionally, as the pharmaceutically acceptable salts thereof. For the purposes of administration and dosing, the compounds or pharmaceutically acceptable salts thereof are collectively referred to as the compounds of the present disclosure.

The compounds of the present disclosure are administered via any suitable route in the form of a pharmaceutical composition appropriate for that route, and at a therapeutically effective dose for the intended treatment. The compounds of the present disclosure may be administered orally, rectally, vaginally, parenterally, or topically.

As used herein, the term "administration" refers to the absorption, ingestion, injection, inhalation, implantation, or other means of introducing the compounds of the present disclosure or the pharmaceutical compositions thereof. The term "treatment" refers to reversing, alleviating, delaying the onset of, or inhibiting the progression of the "pathological condition" described herein (e.g., a disease, disorder, or condition, or one or more signs or symptoms thereof). In some embodiments, treatment may be administered after one or more signs or symptoms of the disease or condition have developed or been observed. In other embodiments, treatment may be performed in the absence of signs or symptoms of the disease or condition. For example, treatment may be given to a susceptible individual before symptom onset (e.g., based on symptom history and/or genetic or other susceptibility factors). Treatment may also be continued after symptom remission, for example, to delay or prevent relapse. As used herein, the terms "disease", "disorder", "condition", and "pathological condition" are used interchangeably.

Those skilled in the art may determine appropriate dosage levels through routine experimentation. The dosage regimen of the compounds of the present disclosure and/or compositions comprising such compounds is based on various factors, including the type, age, weight, sex, and medical condition of the patient; the severity of the condition; the route of administration; and the activity of the specific compound used. Therefore, the dosage regimen may vary widely. For example, the dosage level of the compounds of the present disclosure may be from about 0.001 to about 100 mg/kg per day (i.e., mg per kg of body weight). In some embodiments, the total daily dose of the compounds of the present disclosure, administered either as a single dose or in divided doses, may be from approximately 0.001 to approximately 10 mg/kg. It is not uncommon for administration of the compounds of the present disclosure to be repeated multiple times in a single day.

### Pharmaceutical Composition

In some aspects, the present disclosure relates to a pharmaceutical composition comprising a compound of the present disclosure as provided herein, or a pharmaceutically acceptable salt thereof, and at least one pharmaceutically acceptable carrier or excipient.

As used herein, the term "pharmaceutically acceptable carrier or excipient" refers to a carrier or excipient that may be used in the preparation of a pharmaceutical composition and that is generally safe, non-toxic, and not biologically or otherwise undesirable, and includes carriers or excipients that are acceptable for veterinary use as well as for human pharmaceutical use. As used herein, a pharmaceutically acceptable carrier or excipient comprises one or more of such carriers or excipients. The specific carrier or excipient used will depend upon the manner and purpose of the application of the compound of the present disclosure. The suitable carriers and excipients are well known to those skilled in the art and are described, for example, in Ansel, Howard C. et al., Ansel's Pharmaceutical Dosage Forms and Drug Delivery Systems, Philadelphia: Lippincott, Williams & Wilkins, 2004; Gennaro, Alfonso R. et al., Remington: The Science and Practice of Pharmacy. Philadelphia: Lippincott, Williams & Wilkins, 2000; and Rowe, Raymond C., Handbook of Pharmaceutical Excipients, Chicago: Pharmaceutical Press, 2005. These may include one or more of buffers, stabilizers, surfactants, wetting agents, lubricants, emulsifiers, suspending agents, preservatives, antioxidants, opacifying agents, glidants, processing aids, coloring agents, sweeteners, flavoring agents, diluents, and other known additives to provide an elegant presentation of the drug (i.e., the compound or pharmaceutical composition provided herein) or to aid in the manufacture of the pharmaceutical product (i.e., the drug).

The composition of the present disclosure may be formulated in various forms. These include, for example, liquid, semi-solid, and solid dosage forms such as liquid solutions (e.g., injectable and infusible solutions), dispersions or suspensions, tablets, pills, powders, liposomes, suppositories, and the like. The form will depend on the intended route of administration and therapeutic application.

The pharmaceutical composition of the present disclosure may be prepared using any well-known pharmaceutical techniques (such as effective formulation and administration procedures). The above considerations regarding effective formulation and administration procedures are well known in the art and are described in standard textbooks. For example, the formulations of pharmaceutical products are discussed in Hoover, John E., Remington's Pharmaceutical Sciences, Mack Publishing Co., Easton, Pennsylvania, 1975; Liberman et al., (Ed.), Pharmaceutical Dosage Forms, Marcel Decker, New York, N.Y., 1980; and Kibbe et al., (Ed.), Handbook of Pharmaceutical Excipients, 3rd edition, American Pharmaceutical Association, Washington, 1999.

In yet another aspect, the present disclosure relates to a kit for treating a hyperproliferative disease, such as cancer, comprising a compound of the present disclosure or a pharmaceutically acceptable salt thereof as provided herein, or a pharmaceutical composition comprising a compound of the present disclosure or a pharmaceutically acceptable salt thereof as provided herein, a vessel, and optionally a package insert or label indicating the treatment.

### Treatment Method

In yet another aspect, the present disclosure relates to a method for treating a hyperproliferative disease, such as cancer, in a subject in need thereof. Due to the KIF18A protein inhibitory activity of the compounds of the present disclosure, the method comprises administering to the subject a therapeutically effective amount of a compound of the present disclosure or a pharmaceutically acceptable salt thereof as provided herein.

As used herein, the term "subject in need thereof" refers to a subject who has a hyperproliferative disease, such as cancer, or a subject who is at increased risk of developing a hyperproliferative disease, such as cancer, relative to the general population. In some embodiments, the subject is a homeothermic animal. In some embodiments, the homeothermic animal is a mammal. In some embodiments, the homeothermic animal is a human.

In some embodiments, the cancer includes any one selected from the following: colon cancer, breast cancer, lung cancer, pancreatic cancer, prostate cancer, bladder cancer, head cancer, neck cancer, cervical cancer, and ovarian cancer.

The method of treating a hyperproliferative disease, such as cancer, as described herein may be used as a monotherapy. As used herein, the term "monotherapy" refers to the administration of a single active or therapeutic compound to a subject in need thereof. In some embodiments, the monotherapy involves administering a therapeutically effective amount of a compound of the present disclosure or a pharmaceutically acceptable salt thereof to a subject in need of such treatment.

In yet another aspect, the present disclosure relates to the use of a compound of the present disclosure or a pharmaceutically acceptable salt thereof, as provided herein, for the treatment of a hyperproliferative disease such as cancer.

In yet another aspect, the present disclosure relates to the use of a compound of the present disclosure or a pharmaceutically acceptable salt thereof, as provided herein, in the preparation of a medicament for the treatment of a hyperproliferative disease such as cancer.

### Synthesis

The compounds of the present disclosure may be prepared using general knowledge well known to those skilled in the field of synthetic organic chemistry, through the general and specific methods described below. Such general knowledge can be found in standard reference works, for example, Comprehensive Organic Chemistry (Ed.) by Barton and Ollis, Elsevier; Richard Larock, Comprehensive Organic Transformations: A Guide to Functional Group Preparations, John Wiley and Sons; and Encyclopedia of Reagents for Organic Synthesis, Volumes I-XII, Wiley-Interscience.

The schemes described below are intended to provide a general description of the methods for preparing the compounds of the present disclosure. Some compounds of the present disclosure may comprise one or more chiral centers designated by the stereochemical descriptors (R) or (S). It will be apparent to those skilled in the art that all synthetic transformations may be carried out in a similar manner regardless of whether the material is enantiomerically enriched or racemic. Furthermore, the resolution of the desired optically active materials may be performed at any desired point in the process using well-known methods, such as those described herein and in the chemical literature.

### Embodiments

The present disclosure is further described below with specific embodiments. It should be understood that these embodiments are provided solely for the purpose of illustrating the present disclosure and are not intended to limit the scope of the present disclosure. In the following embodiments, the experimental methods without specified conditions are typically conducted under conventional conditions for such reactions or as recommended by the manufacturer, unless otherwise stated. Unless otherwise stated, percentages and weight fractions are percentages and parts by weight. Unless otherwise stated, the ratios of liquids are the volume ratios.

During synthesis, it may be necessary and/or desirable to protect sensitive or reactive groups on the relevant molecules. This may be achieved by means of conventional protecting groups, such as those described in Protective Groups in Organic Synthesis, 4th edition, by T.W. Greene and P.G.M. Wutts [sic: Wuts], John Wiley and Sons. The protecting groups may optionally be removed at a convenient later stage using methods well known in the art.

The compounds of the present disclosure may be readily prepared according to the following reaction schemes and embodiments or their modifications, using readily available starting materials, reagents, and conventional synthetic procedures. Variants known to those skilled in the art but not described in further detail may also be used in these reactions. Furthermore, other methods for preparing the compounds of the present disclosure, based on the reaction schemes and embodiments described herein, will be apparent to those skilled in the art. All variables are defined as above unless otherwise specified. In general, in the chemical procedures, all reagents and starting materials may be purchased from commercial suppliers or may be readily prepared by those skilled in the art.

The chemical formulas or abbreviations represent the following reagents:
NaH represents sodium cyanide; CoCl₂ [sic: COCl₂] represents phosgene; NaBH₄ represents sodium borohydride; K₂CO₃ represents potassium carbonate; Cu(OAc)₂ represents copper acetate; DMF represents *N,N-dimethylformamide;* CuI represents copper(I) iodide; K₃PO₄ represents potassium phosphate; DIEA represents N,N-diisopropylethylamine; NMP represents N-methylpyrrolidone; DMSO represents dimethyl sulfoxide; LiAlH₄ represents lithium aluminum hydride; NaOH represents sodium hydroxide; DCM represents dichloromethane; PPh₃ represents triphenylphosphine; CBr₄ represents carbon tetrabromide; THF represents tetrahydrofuran; *^{t}*BuOK represents potassium tert-butoxide; TBAI represents tetrabutylammonium iodide; MgCl₂ represents magnesium chloride.

### Embodiment 1: 2-Hydroxy-N-{2'-[6-(1,4-oxazinane-4-yl) pyridin-2-yl]-1'-oxo-2',3'-dihydro-1'H-spiro[cyclohexane-1,4'-isoquinoline]-6'-yl} ethanesulfonamide (EX01)

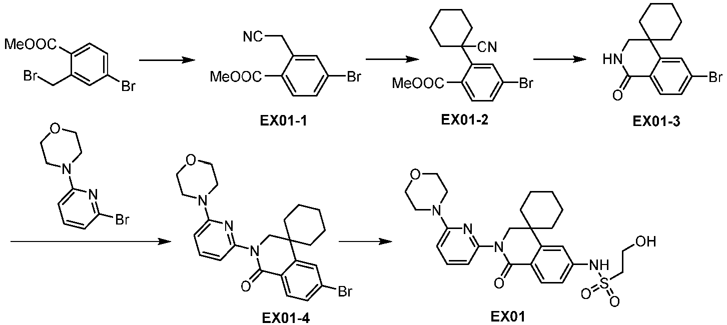

### Step 1:

A mixed solution of potassium cyanide (1.11 g, 17.0 mmol) in water (35 mL) and ethanol (35 mL) was added to a solution of methyl 4-bromo-2-(bromomethyl)benzoate (5.00 g, 16.2 mmol) in 1,4-dioxane (35 mL). The resulting reaction mixture was heated to 100 °C and stirred for 5 hours. Upon completion, the reaction mixture was cooled to room temperature, diluted with water (20 mL), and extracted with ethyl acetate (30 mL × 3). The combined organic layers were washed with saturated brine (30 mL), dried over anhydrous sodium sulfate, and concentrated under reduced pressure. The resulting residue was purified by preparative HPLC to obtain intermediate EX01-1. LCMS [M+H]⁺: 256.0.

### Step 2:

Under heating at 65 °C and nitrogen protection, a solution of intermediate EX01-1 (4.00 g, 20.4 mmol) and 1,5-dibromopentane (2.15 mL, 15.7 mmol) in tetrahydrofuran (10 mL) was slowly added dropwise to a suspension of NaH (3.15 g, 78.7 mmol) in tetrahydrofuran (40 mL). The resulting reaction mixture was stirred at 65 °C for 3 hours. Upon completion, the reaction mixture was cooled to room temperature, diluted with saturated ammonium chloride solution (10 mL), and extracted with ethyl acetate (10 mL × 3). The combined organic layers were washed with saturated brine (20 mL), dried over anhydrous sodium sulfate, and concentrated under reduced pressure. The resulting residue was purified by preparative HPLC to obtain intermediate EX01-2. LCMS [M+H]⁺: 324.1.

### Step 3:

Under cooling at 0 °C and nitrogen protection, NaBH₄ (400 mg, 10.6 mmol) was slowly added to a methanol (20 mL) solution of intermediate EX01-2 (400 mg, 1.241 mmol) and CoCl₂ [sic: COCl₂] (484 mg, 3.724 mmol). The resulting mixture was stirred at 25 °C for 50 hours. Upon completion, the reaction mixture was diluted with water (50 mL) and extracted with ethyl acetate (100 mL × 3). The combined organic layers were washed with saturated brine (100 mL), dried over anhydrous sodium sulfate, and concentrated under reduced pressure. The resulting residue was purified by preparative TLC to obtain intermediate EX01-3. LCMS [M+H]⁺: 296.0.

### Step 4:

A mixture of intermediate EX01-3 (83.0 mg, 0.341 mmol), 2-bromo-6-(4-morpholinyl)pyridine (CAS: 332134-60-8, 100 mg, 0.341 mmol), K₂CO₃ (118 mg, 0.85 mmol), Cu(OAc)₂ (124 mg, 0.68 mmol), and 3,4,7,8-tetramethyl-1,10-phenanthroline (CAS: 1660-93-1, 40.3 mg, 0.171 mmol) in 1,4-dioxane (4 mL) was degassed by nitrogen purging three times. Under nitrogen protection, the resulting mixture was heated to 100 °C and stirred for 8 hours. Upon completion, the reaction mixture was cooled to room temperature, diluted with water (30 mL), and extracted with ethyl acetate (30 mL × 3). The combined organic layers were washed with saturated brine (30 mL), dried over anhydrous sodium sulfate, and concentrated under reduced pressure. The resulting residue was purified by preparative TLC to obtain intermediate EX01-4. LCMS [M+H]⁺: 296.0.

### Step 5:

CuI (14.6 mg, 0.08 mmol), K₃PO₄ (48.8 mg, 0.23 mmol), and (1R,2R)-(-)-N,N'-dimethyl-1,2-cyclohexanediamine (CAS: 68737-65-5, 10.9 mg, 0.08 mmol) were added to a solution of intermediate EX01-4 (35.0 mg, 0.07 mmol) and 2-Hydroxyethane-1-sulfonamide (9.60 mg, 0.077 mmol) in DMF (1 mL). Under nitrogen protection, the mixture was heated to 100 °C and stirred for 10 hours. Upon completion, the reaction mixture was cooled to room temperature, diluted with water (30 mL), and extracted with ethyl acetate (30 mL × 3). The combined organic layers were washed with saturated brine (30 mL), dried over anhydrous sodium sulfate, and concentrated under reduced pressure. The resulting residue was purified by preparative HPLC to obtain product EX01. ¹H NMR (400 MHz, Methanol-*d*₄) δ 8.05 (d, *J =* 8.2 Hz, 1H), 7.64 (t, *J =* 8.1 Hz, 1H), 7.41 (s, 1H), 7.29 (d, *J = 7.8* Hz, 1H), 7.26 (d, *J =* 8.3 Hz, 1H), 6.66 (d, *J =* 8.3 Hz, 1H), 4.26 (s, 2H), 3.97 (t, *J =* 7.0 Hz, 2H), 3.83 (t, *J* = 4.9 Hz, 4H), 3.55 (t, *J =* 4.9 Hz, 4H), 3.43 - 3.36 (m, 2H), 1.83 - 1.26 (m, 10H). LCMS[M+H]⁺: 501.4.

### Embodiment 2: N-{2-[2-(4,4-Difluorohexahydropyridin-1-yl)-6-methylpyrimidin-4-yl]-1-oxo-2,3-dihydro-1H-spiro[isoquinoline-4,6'-spiro[2.5]octane]-6-yl}-2-hydroxyethanesulfonamide (EX02)

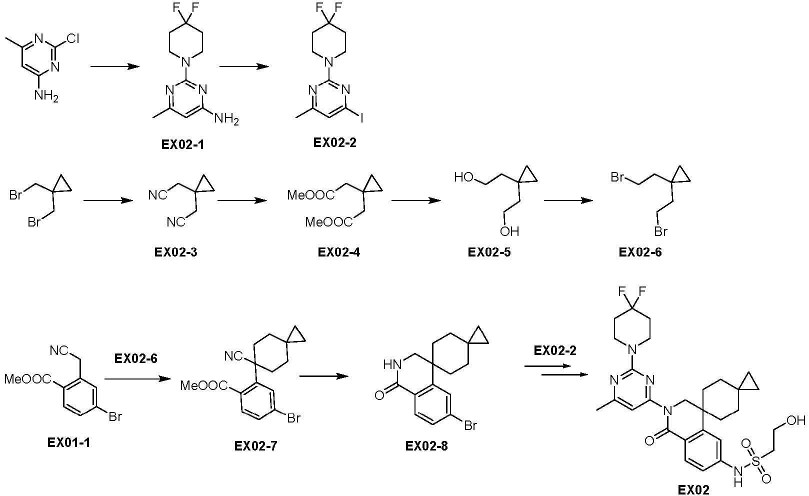

### Step 1:

A solution of 2-chloro-4-amino-6-methylpyrimidine (5.0 g, 34.8 mmol), 4,4-difluoropiperidine hydrochloride (8.23 g, 52.2 mmol), and DIEA (17.2 mL, 104 mmol) in NMP (50 mL) was heated to 140°C and stirred for 30 hours. Upon completion, the reaction mixture was cooled to room temperature, diluted with water (50 mL) and extracted with ethyl acetate (50 mL × 3). The combined organic layers were washed with saturated brine (50 mL), dried over anhydrous sodium sulfate, and concentrated under reduced pressure to obtain intermediate EX02-1. LCMS[M+H]⁺: 229.1.

### Step 2:

A solution of intermediate EX02-1 (500 mg, 2.191 mmol), 3-methyl-1-nitrobutane (1,539.76 mg, 13.143 mmol), and diiodomethane (118.23 mg, 0.438 mmol) in 1,4-dioxane (10 mL) was degassed with nitrogen purging three times. Under nitrogen protection, the resulting reaction mixture was heated to 80°C and stirred for 12 hours. Upon completion, the reaction mixture was concentrated under reduced pressure to obtain intermediate EX02-2. LCMS[M+H]⁺: 340.2.

### Step 3:

Potassium cyanide (11.6 g, 178 mmol) was added to a solution of 1,1-bis(bromomethyl)cyclopropane (15.0 g, 65.8 mmol) in DMSO (200 mL). The mixture was heated to 90°C and stirred for 5 hours. Upon completion, the reaction mixture was cooled to room temperature, diluted with water (20 mL), and extracted with ethyl acetate (30 mL × 3). The combined organic layers were washed with saturated brine (30 mL), dried over anhydrous sodium sulfate, and concentrated under reduced pressure to obtain intermediate EX02-3.

### Step 4:

Sulfuric acid (29.0 mL) was added dropwise to a methanol (66 mL) solution of intermediate EX02-3 (1.00 g, 8.32 mmol) at 0°C. The resulting reaction mixture was degassed with nitrogen purging three times, heated to 65°C, and stirred for 12 hours. Upon completion, the reaction mixture was cooled to room temperature, diluted with water (10 mL), and extracted with ethyl acetate (20 mL × 2). The combined organic layers were washed with saturated brine (30 mL), dried over anhydrous sodium sulfate, and concentrated under reduced pressure. The resulting residue was purified by silica gel column chromatography to obtain intermediate EX02-4. ¹HNMR (400 MHz, Methanol-*d*₄) *δ* ppm 3.66 (s,6H), 2.40 (s,4H), 0.53 (s,4H).

### Step 5:

At 0°C under nitrogen protection, LiAlH₄ (1.60 g, 42.1 mmol) was added to a tetrahydrofuran (50 mL) solution of intermediate EX02-4 (3.80 g, 20.4 mmol). The resulting reaction mixture was stirred at 25°C for 3 hours. Upon completion, the reaction mixture was quenched with water (20 mL) and 10% NaOH solution (5 mL), and extracted with ethyl acetate (100 mL × 3). The combined organic layers were washed with saturated brine (100 mL), dried over anhydrous sodium sulfate, and concentrated under reduced pressure to obtain intermediate EX02-5. ¹HNMR (400 MHz, Methanol-*d*₄) *δ* ppm 3.66 - 3.72 (m,4H), 1.56 (t, *J =* 6.4 Hz, 4H), 0.33 (s,4H).

### Step 6:

At 0°C under nitrogen protection, PPh₃ (1.60 g, 42.1 mmol) and CBr₄ (509 mg, 1.53 mmol) were successively added to a DCM (8 mL) solution of intermediate EX02-5 (100 mg, 0.768 mmol). The resulting reaction mixture was stirred at 25°C for 12 hours. Upon completion, the reaction mixture was concentrated under reduced pressure and purified by silica gel column chromatography to obtain intermediate EX02-6.

### Step 7:

At 0 °C under nitrogen protection, *^{t}*BuOK (883 mg, 7.87 mmol) was added to a THF (8 mL) solution of intermediate EX01-1 (800 mg, 3.15 mmol). After stirring the resulting mixture for 30 minutes, a tetrahydrofuran (7 mL) solution of TBAI (1.16 g, 3.15 mmol) and EX02-6 (558 mg, 2.16 mmol) was added. The resulting mixture was stirred for 2 hours at 25 °C. Upon completion, the resulting reaction mixture was diluted with water (50 mL) and extracted with ethyl acetate (60 mL × 3). The combined organic layers were washed with saturated brine (30 mL), dried over anhydrous sodium sulfate, and concentrated under reduced pressure. The resulting residue was purified by silica gel column chromatography to obtain intermediate EX02-7. LCMS [M+H]⁺: 350.0.

### Step 8:

The intermediate EX02-8 was prepared using a method similar to Step 3 of Embodiment 1 (EX01). Wherein, the intermediate EX01-2 was replaced with the intermediate EX02-7. LCMS [M+H]⁺: 322.2.

### Step 9:

The product EX02 was prepared using a method similar to Steps 4-5 of Embodiment 1 (EX01). Wherein, the intermediate EX01-3 and 2-bromo-6-(4-morpholinyl)pyridine were replaced with the intermediate EX02-8 and the intermediate EX02-2, respectively. ¹H NMR (400 MHz, Methanol-*d*₄) δ 8.06 (d, *J =* 8.4 Hz, 1H), 7.53 (s, 1H), 7.40 (d, *J =* 2.2 Hz, 1H), 7.26 (dd, *J =* 8.6, 2.1 Hz, 1H), 4.47 (s, 2H), 4.02 (t, *J =* 5.8 Hz, 4H), 3.95 (t, *J* = 6.2 Hz, 2H), 3.37 (t, *J =* 6.3 Hz, 2H), 2.37 (s, 3H), 2.15 - 1.90 (m, 8H), 1.72 (d, *J* = 13.0 Hz, 2H), 0.89 (d, *J =* 13.4 Hz, 2H), 0.34 (s, 4H). LCMS[M+H]⁺: 576.8.

### Embodiment 3: N-{2'-[2-(4,4-difluorohexahydropyridin-1-yl)-6-methylpyrimidin-4-yl]-1,1-dimethyl-1'-oxo-2',3'-dihydro-1'H-spiro[cyclohexane-4,4'-isoquinoline]-6'-yl}-2-hydroxyethanesulfonamide (EX03)

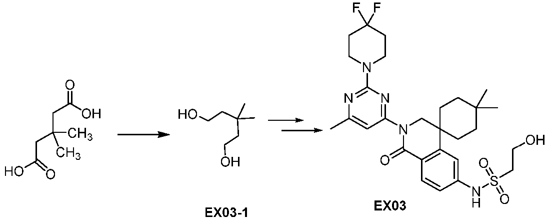

### Step 1:

At 0°C under nitrogen protection, a tetrahydrofuran solution of 3,3-dimethylglutaric acid (5.00 g, 31.2 mmol) was added to a tetrahydrofuran (20 mL) solution of LiAlH₄ (2.49 g, 65.5 mmol). The resulting mixture was heated to 65 °C and stirred for 5 hours. Upon completion, the reaction mixture was cooled to room temperature, quenched with water (20 mL) and 10% NaOH solution (5 mL), and extracted with ethyl acetate (100 mL × 3). The combined organic layers were washed with saturated brine (100 mL), dried over anhydrous sodium sulfate, and concentrated under reduced pressure to obtain intermediate EX03-1. ¹H NMR (400 MHz, CDCl₃) δ 3.62 (q, *J =* 7.6 Hz, 4H), 1.44 - 1.56 (m, 4H), 0.84 - 0.92 (m, 6H).

### Step 2:

The product EX03 was prepared using a method similar to Steps 6-9 of Embodiment 2 (EX02). Wherein, the intermediate EX02-5 was replaced with the intermediate EX03-1. ¹H NMR (400 MHz, Methanol-*d*₄) δ 8.06 (d, *J* = 8.5 Hz, 1H), 7.52 (s, 1H), 7.41 (d, *J =* 2.2 Hz, 1H), 7.26 (dd, *J =* 8.5, 2.2 Hz, 1H), 4.38 (s, 2H), 4.01 (t, *J =* 5.8 Hz, 4H), 3.95 (t, *J =* 6.2 Hz, 2H), 3.37 (t, *J =* 6.2 Hz, 2H), 2.37 (s, 3H), 2.08 - 1.91 (m, 6H), 1.64 - 1.51 (m, 4H), 1.45 - 1.36 (m, 2H), 1.05 (s, 3H), 1.00 (s, 3H). LCMS[M+H]⁺: 578.4.

### Embodiment 4: 2-Hydroxy-N-(2-{6-[(2R)-2-methyl-1,4-oxazinane-4-yl]pyridin-2-yl}-1-oxo-2,3-dihydro-1H-spiro[isoquinoline-4,6'-spiro[2.5]octane]-6-yl)ethanesulfonamide (EX04)

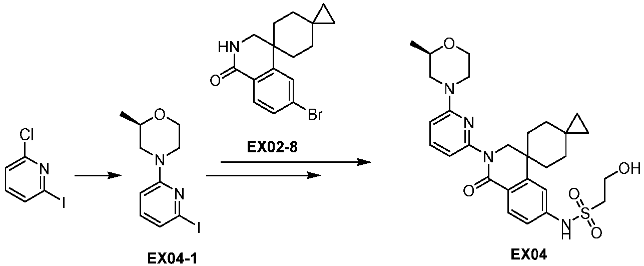

### Step 1:

K₂CO₃ (6.93 g, 50.1 mmol) was added to a solution of 2-Chloro-6-iodopyridin-3-amine (3.0 g, 12.5 mmol) and (*R*)-2-methylmorpholine hydrochloride (CAS: 168038-14-0, 2.59 g, 18.7 mmol) in DMF (30 mL). The resulting mixture was heated to 100 °C and stirred for 12 hours. Upon completion, the reaction mixture was cooled to room temperature, diluted with water (100 mL), and extracted with ethyl acetate (30 mL × 3). The combined organic layers were washed with saturated brine (30 mL), dried over anhydrous sodium sulfate, and concentrated under reduced pressure. The resulting residue was isolated and purified by preparative HPLC to obtain intermediate EX04-1. LCMS [M+H]⁺: 305.0.

### Step 2:

The product EX04 was prepared using a method similar to Steps 4-5 of Embodiment 1 (EX01). Wherein, the intermediate EX01-3 and 2-bromo-6-(4-morpholinyl)pyridine were replaced with the intermediate EX02-8 and the intermediate EX04-1, respectively. ¹H NMR (400 MHz, Methanol-*d*₄) δ 8.05 (d, *J =* 8.5 Hz, 1H), 7.63 (t, *J =* 8.0 Hz, 1H), 7.42 (d, *J =* 2.2 Hz, 1H), 7.38 (d, *J =* 7.8 Hz, 1H), 7.27 (dd, *J* = 8.4*,* 2.1 Hz, 1H), 6.65 (d, *J =* 8.3 Hz, 1H), 4.42 - 4.30 (m, 2H), 4.24 (d, *J =* 12.7 Hz, 1H), 4.08 - 3.99 (m, 2H), 3.97 (t, *J =* 6.3 Hz, 2H), 3.77 - 3.68 (m, 2H), 3.38 (t, *J =* 6.4 Hz, 2H), 2.96 (td, *J =* 12.2, 3.1 Hz, 1H), 2.61 (dd, *J =* 12.8, 10.4 Hz, 1H), 2.14 (t, *J =* 14.3 Hz, 2H), 1.98 (t, *J =* 13.1 Hz, 2H), 1.81 (d, *J =* 13.1 Hz, 2H), 1.27 (d, *J =* 6.2 Hz, 3H), 0.91 (d, *J =* 13.3 Hz, 2H), 0.36 (s, 4H). LCMS[M+H]⁺: 541.4.

### Embodiment 5: N-{2-[1-(3,3-Difluorocyclobutyl)-6-oxo-1,2-diazacyclohexanecarbonate-3-yl]-1-oxo-2,3-dihydro-1H-spiro[isoquinoline-4,6'-spiro[2.5]octane]-6-yl}-2-hydroxyethanesulfonamide (EX05)

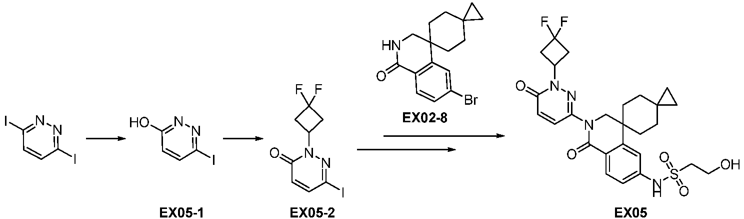

Step 1: An aqueous solution (50 mL) of 3,6-diiodopyridazine (2.0 g, 6.03 mmol) and NaOH (3.33 g, 83.2 mmol) was heated to 100°C and stirred for 2 hours. Upon completion, the reaction mixture was cooled to room temperature, filtered, and the filtrate was concentrated under reduced pressure to obtain intermediate EX05-1. LCMS[M+H]⁺: 223.2.

### Step 2:

3-bromo-1,1-difluorocyclobutane (CAS: 1310729-91-9, 277.3 mg, 1.62 mmol) and K₂CO₃ (560.3 mg, 4.05 mmol) were added to a DMF (5 mL) solution of intermediate EX05-1 (300 mg, 1.35 mmol). The resulting mixture was heated to 80 °C and stirred for 15 hours. Upon completion, the reaction mixture was cooled to room temperature, diluted with water (20 mL), and extracted with ethyl acetate (20 mL × 3). The combined organic layers were washed with saturated brine (10 mL), dried over anhydrous sodium sulfate, and concentrated under reduced pressure. The resulting residue was isolated and purified by preparative TLC to obtain intermediate EX05-2. LCMS[M+H]⁺: 313.1.

### Step 3:

The product EX04 was prepared using a method similar to Steps 4-5 of Embodiment 1 (EX01). Wherein, the intermediate EX01-3 and 2-bromo-6-(4-morpholinyl)pyridine were replaced with the intermediate EX02-8 and the intermediate EX05-2, respectively. ¹H NMR (400 MHz, Methanol-*d*₄) δ 8.09 - 8.01 (m, 2H), 7.46 (d, *J =* 2.1 Hz, 1H), 7.30 (dd, *J =* 8.5, 2.1 Hz, 1H), 7.01 (d, *J =* 9.9 Hz, 1H), 5.50 - 5.39 (m, 1H), 4.24 (s, 2H), 3.97 (t, *J =* 6.2 Hz, 2H), 3.40 (t, *J =* 6.2 Hz, 3H), 3.19 - 3.07 (m, 4H), 2.16 - 1.96 (m, 4H), 1.86 (d, *J =* 11.9 Hz, 2H), 0.97 (d, *J =* 12.6 Hz, 2H), 0.38 (s, 4H)LCMS[M+H]⁺: 549.1.

### Embodiment 6: Biological example: KIF18A enzyme inhibition activity assay

1) The required reagents, materials, equipment, and consumables are listed in Table 1 below.

**Table 1**

| Materials and Reagents | Supplier |
|---|---|
| HEPES buffer | Life Technologies |
| MgCl₂ | Sigma |
| Taxol (paclitaxel) | Cytoskeleton |
| ADP-Glo kit | Promega |
| ATP (adenosine triphosphate) | Promega |
| Porcine tubulin | Cytoskeleton |
| DTT (dithiothreitol) | Sigma |
| 384-well polypropylene plate | Labcyte |
| 96-well polypropylene plate | Nunc |
| Vibrator | Thermo |
| Centrifuge | Eppendorf |
| EnVision multi-mode microplate reader | PerkinElmer |

2) Steps:
2.1 Preparation of enzyme protein buffer solution
2.2 Activity screening
   a) 40 µL of compound solution was added to a 384-well dilution plate;
   b) A 3-fold serial dilution was performed to obtain 10 concentration points;
   c) 0.1 µL of compound solution was transferred into a 384-well plate;
   d) 5 µL of enzyme reaction solution was added and centrifuged at 1,000 RPM for 1 minute;
   e) The reaction took place at 25°C for 15 minutes;
   f) 5 µL of ATP reaction solution was added;
   g) The reaction took place at 25°C for 60 minutes;
   h) 10 µL of ADP-Glo reagent was added and centrifuged at 1,000 RPM for 1 minute;
   i) The reaction took place at 25°C for 60 minutes;
   j) 20 µL of detection reagent was added and centrifuged at 1,000 RPM for 1 minute;
   k) The reaction took place at 25°C for 60 minutes;
   l) The Envision 2104 plate reader was used to measure fluorescence signal.

3) Data analysis
3.1 Fluorescence signal values were obtained from the readings of each well
3.2 Calculation of IC₅₀
Concentration-response curve fitting and IC₅₀ determination were performed using GraphPad Prism software with a four-parameter logistic regression model.
4) The activity results are shown in Table 2.

**Table 2**

| Compound No. | KIF18A IC₅₀ (nM) |
|---|---|
| EX01 | 2,034 |
| EX02 | 38 |
| EX03 | 44 |
| EX04 | 107 |
| EX05 | 8.2 |

All literature mentioned in the present disclosure is cited as a reference in the present application, just like each piece of literature being cited separately as a reference. It should also be understood that, after reading the above description of the contents of the present disclosure, those skilled in the art may make various changes or modifications to the present disclosure, and such equivalents similarly fall within the scope defined by the claims appended to the present application.

## Claims

1. A compound represented by Formula II, or a pharmaceutically acceptable salt thereof, 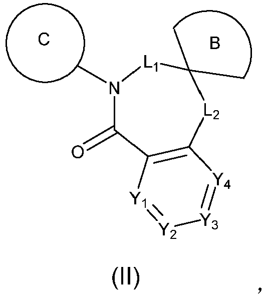
wherein ring C is selected from a C₆₋₁₂ aryl group, a 5- to 12-membered heteroaryl group, a C₃₋₁₈ cycloalkyl group, and a 3- to 18-membered heterocyclyl group, wherein the C₆₋₁₂ aryl group, 5- to 12-membered heteroaryl group, C₃₋₁₈ cycloalkyl group, or 3- to 18-membered heterocyclyl group is optionally substituted with 1, 2, 3, or 4 R_{c} groups;
R_{c} is independently selected from a halogen, a hydroxyl group, a cyano group, SF₅, -S(=O)₂R, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group, wherein the C₁₋₁₂ alkyl group, C₂₋₁₂ alkenyl group, C₂₋₁₂ alkynyl group, C₁₋₁₂ heteroalkyl group, C₂₋₁₂ heteroalkenyl group, C₂₋₁₂ heteroalkynyl group, C₆₋₁₂ aryl group, 5- to 12-membered heteroaryl group, C₃₋₁₂ cycloalkyl group, or 3- to 12-membered heterocyclyl group is optionally substituted with 1, 2, 3, or 4 R_{cc} groups;
alternatively, two R_{c} groups may be attached to the same carbon atom and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{cc} groups;
alternatively, two R_{c} groups may be attached to different carbon atoms and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{cc} groups;
L₁ is selected from a single bond, O, S, N(R_{L}), a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, and a C₂₋₁₂ heteroalkynyl group, wherein the N(R_{L}), C₁₋₁₂ alkyl group, C₂₋₁₂ alkenyl group, C₂₋₁₂ alkynyl group, C₁₋₁₂ heteroalkyl group, C₂₋₁₂ heteroalkenyl group, or C₂₋₁₂ heteroalkynyl group is optionally substituted with 1, 2, 3, or 4 R_{L1} groups;
L₂ is selected from a single bond, O, S, N(R_{L}), N(R_{L})C(=O), a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, and a C₂₋₁₂ heteroalkynyl group, wherein the N(R_{L}), N(R_{L})C(=O), C₁₋₁₂ alkyl group, C₂₋₁₂ alkenyl group, C₂₋₁₂ alkynyl group, C₁₋₁₂ heteroalkyl group, C₂₋₁₂ heteroalkenyl group, or C₂₋₁₂ heteroalkynyl group is optionally substituted with 1, 2, 3, or 4 R_{L2} groups;
ring B is selected from a C₃₋₁₂ cycloalkyl group and a 3- to 12-membered heterocyclyl group, wherein the C₃₋₁₂ cycloalkyl group or 3- to 12-membered heterocyclyl group is optionally substituted with 1, 2, 3, or 4 R_{b} groups;
R_{b} is independently selected from a halogen, a hydroxyl group, a cyano group, SF₅, a C₁₋₁₂ alkyl group, a C₂₋₁₂ alkenyl group, a C₂₋₁₂ alkynyl group, a C₂₋₁₂ heteroalkenyl group, a C₂₋₁₂ heteroalkynyl group, a C₁₋₁₂ heteroalkyl group, a C₆₋₁₂ aryl group, a 5- to 12-membered heteroaryl group, a C₃₋₁₂ cycloalkyl group, and a 3- to 12-membered heterocyclyl group;
alternatively, two R_{b} groups are attached to the same carbon atom to form =C(R_{bbb})₂ or =O;
alternatively, two R_{b} groups may be attached to the same carbon atom and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{bb} groups;
alternatively, two R_{b} groups may be attached to different carbon atoms and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{bb} groups;
Y₁ is selected from N and C(R_{Y1});
Y₂ is selected from N and C(R_{Y2});
Y₃ is selected from N and C(R_{Y3});
Y₄ is selected from N and C(R_{Y4});
R_{Y1}, R_{Y2}, R_{Y3}, and R_{Y4} are each independently selected from hydrogen, a halogen, a hydroxyl group, a cyano group, SF₅, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group, wherein the C₁₋₁₂ alkyl group, C₂₋₁₂ alkenyl group, C₂₋₁₂ alkynyl group, C₁₋₁₂ heteroalkyl group, C₂₋₁₂ heteroalkenyl group, C₂₋₁₂ heteroalkynyl group, C₆₋₁₂ aryl group, 5- to 12-membered heteroaryl group, C₃₋₁₂ cycloalkyl group, or 3- to 12-membered heterocyclyl group is optionally substituted with 1, 2, 3, or 4 R_{Y} groups;
alternatively, R_{Y1}, R_{Y2}, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{Y} groups;
alternatively, R_{Y2}, R_{Y3}, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{Y} groups;
alternatively, R_{Y3}, R_{Y4}, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{Y} groups;
R_{Y} is independently selected from a halogen, a hydroxyl group, a cyano group, SF₅, -S(=O)₂R, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group, wherein the C₁₋₁₂ alkyl group, C₂₋₁₂ alkenyl group, C₂₋₁₂ alkynyl group, C₁₋₁₂ heteroalkyl group, C₂₋₁₂ heteroalkenyl group, C₂₋₁₂ heteroalkynyl group, C₆₋₁₂ aryl group, 5- to 12-membered heteroaryl group, C₃₋₁₂ cycloalkyl group, or 3- to 12-membered heterocyclyl group is optionally substituted with 1, 2, 3, or 4 R' groups;
R is independently selected from a halogen, a hydroxyl group, a cyano group, SF₅, -S(=O)₂R, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group, wherein the C₁₋₁₂ alkyl group, C₂₋₁₂ alkenyl group, C₂₋₁₂ alkynyl group, C₁₋₁₂ heteroalkyl group, C₂₋₁₂ heteroalkenyl group, C₂₋₁₂ heteroalkynyl group, C₆₋₁₂ aryl group, 5- to 12-membered heteroaryl group, C₃₋₁₂ cycloalkyl group, or 3- to 12-membered heterocyclyl group is optionally substituted with 1, 2, 3, or 4 R' groups;
R_{bb}, R_{cc}, R_{L}, R_{L1}, R_{L2}, and R' are each independently selected from a halogen, a hydroxyl group, a cyano group, SF₅, -S(=O)₂R, a C₁₋₁₂ alkyl group, a C₂₋₁₂ alkenyl group, a C₂₋₁₂ alkynyl group, a C₁₋₁₂ heteroalkyl group, a C₂₋₁₂ heteroalkenyl group, a C₂₋₁₂ heteroalkynyl group, a C₆₋₁₂ aryl group, a 5- to 12-membered heteroaryl group, a C₃₋₁₂ cycloalkyl group, and a 3- to 12-membered heterocyclyl group;
alternatively, two R_{cc} groups are attached to the same carbon atom to form =C(R_{ccc})₂ or =O;
alternatively, two R' groups are attached to the same carbon atom to form =C(R")₂ or =O;
R_{bbb}, R_{ccc}, and R" are each independently selected from a halogen, a hydroxyl group, a cyano group, SF₅, a C₁₋₁₂ alkyl group, a C₂₋₁₂ alkenyl group, a C₂₋₁₂ alkynyl group, a C₂₋₁₂ heteroalkenyl group, a C₂₋₁₂ heteroalkynyl group, a C₁₋₁₂ heteroalkyl group, a C₆₋₁₂ aryl group, a 5- to 12-membered heteroaryl group, a C₃₋₁₂ cycloalkyl group, and a 3- to 12-membered heterocyclyl group;
the heteroalkyl group, heteroalkenyl group, heteroalkynyl group, heterocyclyl group, and heteroaryl group each comprise 1, 2, 3, or 4 heteroatoms or heteroatomic groups independently selected from O, NH, S, C(=O), C(=O)O, S(=O), S(=O)₂, S(=O)(=NH), and N.

2. The compound according to Claim 1, or a pharmaceutically acceptable salt thereof, wherein ring C is selected from a phenyl group, a 5- to 6-membered monocyclic heteroaryl group, a saturated, partially saturated, or unsaturated fused bicyclic ring system composed of two 5- to 6-membered rings and comprising 0, 1, 2, or 3 N atoms and 0 or 1 atom selected from O and S, and a saturated, partially saturated, or unsaturated fused tricyclic ring system composed of three 5- to 6-membered rings and comprising 0, 1, 2, or 3 N atoms and 0 or 1 atom selected from O and S, wherein the phenyl group, the 5- to 6-membered monocyclic heteroaryl group, the fused bicyclic ring system composed of two 5- to 6-membered rings, and the fused tricyclic ring system composed of three 5- to 6-membered rings are each optionally substituted with 1, 2, 3, or 4 R_{c} groups;
optionally, ring C is selected from 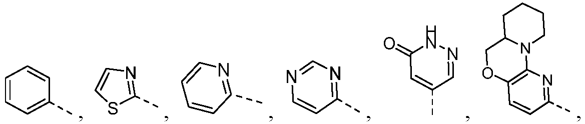 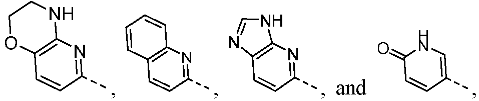 wherein the 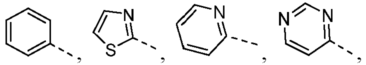 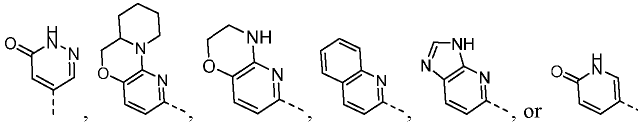 is optionally substituted with 1, 2, 3, or 4 R_{c} groups;
optionally, R_{c} is independently selected from a halogen, a hydroxyl group, a cyano group, SF₅, - S(=O)₂R, a C₁₋₆ alkyl group, a C₂₋₆ alkenyl group, a C₂₋₆ alkynyl group, a C₁₋₆ heteroalkyl group, a C₂₋₆ heteroalkenyl group, a C₂₋₆ heteroalkynyl group, a phenyl group, a 5- to 6-membered heteroaryl group, a C₃₋₆ cycloalkyl group, and a 3- to 6-membered heterocyclyl group, wherein the C₁₋₆ alkyl group, C₂₋₆ alkenyl group, C₂₋₆ alkynyl group, C₁₋₆ heteroalkyl group, C₂₋₆ heteroalkenyl group, C₂₋₆ heteroalkynyl group, phenyl group, 5- to 6-membered heteroaryl group, C₃₋₆ cycloalkyl group, or 3- to 6-membered heterocyclyl group is optionally substituted with 1, 2, 3, or 4 R_{cc} groups;
optionally, R_{c} is independently selected from a halogen, a hydroxyl group, a cyano group, SF₅, - S(=O)₂R, a C₁₋₃ alkyl group, a C₁₋₃ alkoxy group, a 6-membered heteroaryl group, and a C₄₋₆ cycloalkyl group, wherein the C₁₋₃ alkyl group or C₁₋₃ alkoxy group is optionally substituted with 1, 2, 3, or 4 R_{cc} groups;
optionally, R_{cc} is independently selected from a halogen, a hydroxyl group, a cyano group, SF₅, - S(=O)₂R, a C₁₋₆ alkyl group, a C₂₋₆ alkenyl group, a C₂₋₆ alkynyl group, a C₁₋₆ heteroalkyl group, a C₂₋₆ heteroalkenyl group, a C₂₋₆ heteroalkynyl group, a phenyl group, a 5- to 6-membered heteroaryl group, a C₃₋₆ cycloalkyl group, and a 3- to 6-membered heterocyclyl group;
optionally, R_{cc} is independently selected from a halogen, a hydroxyl group, a cyano group, SF₅, - S(=O)₂R, a C₁₋₃ alkyl group, a C₁₋₃ alkoxy group, a 6-membered heteroaryl group, and a C₄₋₆ cycloalkyl group; alternatively, two Rcc groups are attached to the same carbon atom to form =CF₂ or =O;
optionally, ring C is selected from

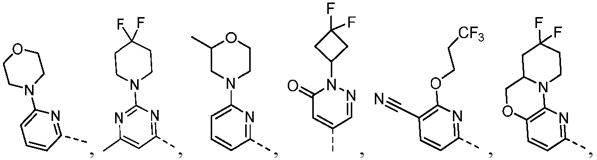

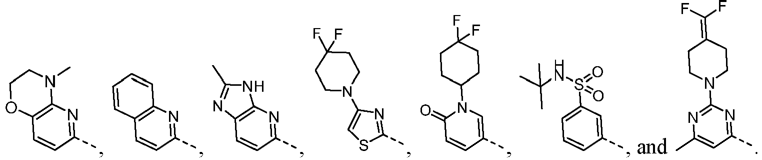

3. The compound according to Claim 1, or a pharmaceutically acceptable salt thereof, wherein ring B is selected from a C₃₋₆ cycloalkyl group and a 3- to 6-membered heterocyclyl group, and wherein the C₃₋₆ cycloalkyl group or 3- to 6-membered heterocyclyl group is optionally substituted with 1, 2, 3, or 4 R_{b} groups;
optionally, ring B is selected from a C₃₋₆ cycloalkyl group and a 5- to 6-membered heterocycloalkyl group, wherein the C₃₋₆ cycloalkyl group or 5- to 6-membered heterocycloalkyl group is optionally substituted with 1, 2, 3, or 4 R_{b} groups;
optionally, R_{b} is independently selected from a halogen, a hydroxyl group, a cyano group, SF₅, a C₁₋₆ alkyl group, a C₂₋₆ alkenyl group, a C₂₋₆ alkynyl group, a C₁₋₆ heteroalkyl group, a C₂₋₆ heteroalkenyl group, a C₂₋₆ heteroalkynyl group, a phenyl group, a 5- to 9-membered heteroaryl group, a C₃₋₆ cycloalkyl group, and a 3- to 9-membered heterocyclyl group;
optionally, two R_{b} groups are attached to the same carbon atom to form =C(R_{bbb})₂ or =O;
optionally, two R_{b} groups are attached to the same carbon atom to form =C(F)₂ or =O;
optionally, two R_{b} groups may be attached to the same carbon atom and, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{bb} groups;
optionally, two R_{b} groups may be attached to the same carbon atom and, together with the carbon to which they are attached, form a C₃₋₆ cycloalkyl ring or a 3- to 6-membered heterocyclic ring, wherein the C₃₋₆ cycloalkyl ring or 3- to 6-membered heterocyclic ring is optionally substituted with 1, 2, 3, or 4 R_{bb} groups;
optionally, two R_{b} groups may be attached to different carbon atoms and, together with the carbon to which they are attached, form a C₃₋₆ cycloalkyl ring or a 3- to 6-membered heterocyclic ring, wherein the C₃₋₆ cycloalkyl ring or 3- to 6-membered heterocyclic ring is optionally substituted with 1, 2, 3, or 4 R_{bb} groups;
optionally, R_{bb} is independently selected from a halogen, a hydroxyl group, a cyano group, SF₅, - S(=O)₂R, a C₁₋₆ alkyl group, a C₂₋₆ alkenyl group, a C₂₋₆ alkynyl group, a C₁₋₆ heteroalkyl group, a C₂₋₆ heteroalkenyl group, a C₂₋₆ heteroalkynyl group, a phenyl group, a 5- to 9-membered heteroaryl group, a C₃₋₆ cycloalkyl group, and a 3- to 6-membered heterocyclyl group;
optionally, R_{bbb} is independently selected from a halogen, a hydroxyl group, a cyano group, SF₅, and a C₁₋₆ alkyl group;
optionally, ring B is selected from 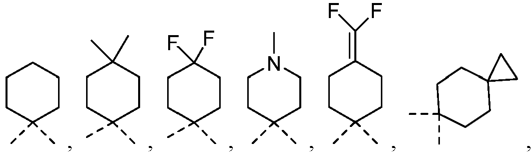 and

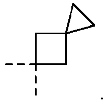

4. The compound according to Claim 1, or a pharmaceutically acceptable salt thereof, wherein R_{Y1}, R_{Y2}, and R_{Y4} are each independently selected from hydrogen, a halogen, a hydroxyl group, a cyano group, SF₅, a C₁₋₆ alkyl group, a C₁₋₆ alkoxy group, a C₁₋₆ alkylamino group, and a C₁₋₆ alkylthio group, wherein the C₁₋₆ alkyl group, C₁₋₆ alkoxy group, C₁₋₆ alkylamino group, or C₁₋₆ alkylthio group is optionally substituted with 1, 2, 3, or 4 R_{Y} groups;
R_{Y3} is independently selected from Z-R_{Y3a}, wherein Z is selected from a single bond, -C₀₋₆ alkyl-S-C₀₋₆ alkyl-, -C₀₋₆ alkyl-S(=O)-C₀₋₆ alkyl-, -C₀₋₆ alkyl-S(=O)₂-C₀₋₆ alkyl-, -C₀₋₆ alkyl-NR_{Y3b}-C₀₋₆ alkyl-, -C₀₋₆ alkyl-NR_{Y3b}-S(=O)₂-C₀₋₆ alkyl-, -C₀₋₆ alkyl-S(=O)₂-NR_{Y3b}-C₀₋₆ alkyl-, -C₀₋₆ alkyl-O-C₀₋₆ alkyl-, -C₀₋₆ alkyl-C(=O)-C₀₋₆ alkyl-, -C₀₋₆ alkyl-C(=O)-O-C₀₋₆ alkyl-, -C₀₋₆ alkyl-C(=O)-NR_{Y3b}-C₀₋₆ alkyl-, -C₀₋₆ alkyl-S(=O)(=NH)-, - N=S(=O)-, -(C=O)-, and -C(=N-OH)-, wherein the -C₀₋₆ alkyl-S-C₀₋₆ alkyl-, -C₀₋₆ alkyl-S(=O)-C₀₋₆ alkyl-, - C₀₋₆ alkyl-S(=O)₂-C₀₋₆ alkyl-, -C₀₋₆ alkyl-NR_{Y3b}-C₀₋₆ alkyl-, -C₀₋₆ alkyl-NR_{Y3b}-S(=O)₂-C₀₋₆ alkyl-, -C₀₋₆ alkylS(=O)₂-NR_{Y3b}-C₀₋₆ alkyl-, -C₀₋₆ alkyl-O-C₀₋₆ alkyl-, -C₀₋₆ alkyl-C(=O)-C₀₋₆ alkyl-, -C₀₋₆ alkyl-C(=O)-O-C₀₋₆ alkyl-, -C₀₋₆ alkyl-C(=O)-NR_{Y3b}-C₀₋₆ alkyl-, or -C₀₋₆ alkyl-S(=O)(=NH)- is optionally substituted with 1, 2, 3, or 4 R_{Y} groups;
R_{Y3b} is independently selected from hydrogen, a halogen, a hydroxyl group, a cyano group, SF₅, and a C₁₋₆ alkyl group;
R_{Y3a} is independently selected from hydrogen, a halogen, a hydroxyl group, a cyano group, SF₅, a C₁₋₆ alkyl group, a saturated, partially saturated, or unsaturated monocyclic ring composed of 3, 4, 5, 6, or 7 members, or a bicyclic ring composed of 8, 9, 10, 11, or 12 members, comprising 0, 1, 2, or 3 N atoms and 0 or 1 atom selected from O and S, wherein the monocyclic ring or bicyclic ring is optionally substituted with 1, 2, 3, or 4 R' groups;
optionally, Z is selected from a single bond, -NH-S(=O)₂-C₀₋₄ alkyl-, -NH-C₀₋₄ alkyl-, -S(=O)₂-NH-C₀₋₄ alkyl-, and -C₀₋₄ alkyl-S(=O)(=NH)-;
optionally, R_{Y3a} is independently selected from hydrogen, a halogen, a hydroxyl group, a cyano group, SF₅, a C₁₋₆ alkyl group, a C₃₋₆ cycloalkyl group, and a 5- to 6-membered heterocyclyl group, wherein the C₁₋₆ alkyl group, C₃₋₆ cycloalkyl group, or 5- to 6-membered heterocyclyl group is optionally substituted with 1, 2, 3, or 4 R' groups;
optionally, R' is independently selected from hydrogen, a halogen, a hydroxyl group, a cyano group, SF₅, and a C₁₋₆ alkyl group; alternatively, two R' groups are attached to the same carbon atom to form =C(F)₂ or =O;
optionally, R_{Y3} is independently selected from

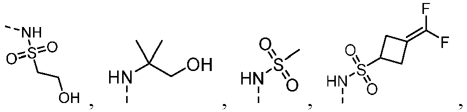

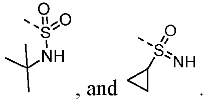

5. The compound according to Claim 1, or a pharmaceutically acceptable salt thereof, wherein L₁ is selected from a single bond, a C₁₋₆ alkyl group, a C₂₋₆ alkenyl group, a C₂₋₆ alkynyl group, a C₁₋₆ heteroalkyl group, a C₂₋₆ heteroalkenyl group, and a C₂₋₆ heteroalkynyl group, wherein the C₁₋₆ alkyl group, C₂₋₆ alkenyl group, C₂₋₆ alkynyl group, C₁₋₆ heteroalkyl group, C₂₋₆ heteroalkenyl group, or C₂₋₆ heteroalkynyl group is optionally substituted with 1, 2, 3, or 4 R_{L1} groups.
optionally, L₁ is selected from a single bond, -CH₂- and -CH₂CH₂-, wherein the -CH₂- or -CH₂CH₂- is optionally substituted with 1 or 2 R_{L1} groups;
optionally, L₂ is selected from a single bond, O, S, N(R_{L}), N(R_{L})C(=O), a C₁₋₆ alkyl group, a C₂₋₆ alkenyl group, a C₂₋₆ alkynyl group, a C₁₋₆ heteroalkyl group, a C₂₋₆ heteroalkenyl group, and a C₂₋₆ heteroalkynyl group, wherein the N(R_{L}), N(R_{L})C(=O), C₁₋₆ alkyl group, C₂₋₆ alkenyl group, C₂₋₆ alkynyl group, C₁₋₆ heteroalkyl group, C₂₋₆ heteroalkenyl group, or C₂₋₆ heteroalkynyl group is optionally substituted with 1, 2, 3, or 4 R_{L2} groups;
optionally, L₂ is selected from a single bond, O, S, -CH₂- and -CH₂CH₂-, wherein the -CH₂- or - CH₂CH₂- is optionally substituted with 1 or 2 R_{L1} groups.

6. The compound according to Claims 1 to 5, or a pharmaceutically acceptable salt thereof, wherein the compound has the following structure: 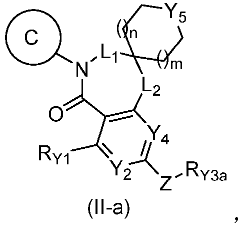
wherein, m is selected from 0, 1, 2, or 3;
n is selected from 0, 1, 2, or 3;
Y₅ is selected from NR_{Y5} and C(R₂)(R₃);
R_{Y5}, R₂, and R₃ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 R_{Y5aa} groups;
alternatively, R₂, R₃, and the carbon to which they are attached form a saturated, partially unsaturated, or unsaturated 5- to 7-membered monocyclic ring, or a saturated, partially unsaturated, or unsaturated 9- to 15-membered bicyclic ring, wherein the monocyclic ring or bicyclic ring is optionally substituted with 1, 2, 3, or 4 R_{Y5aa} groups;
R_{Y5aa} is independently selected from a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group.

7. The compound according to Claims 1 to 5, or a pharmaceutically acceptable salt thereof, wherein the compound has the following structure: 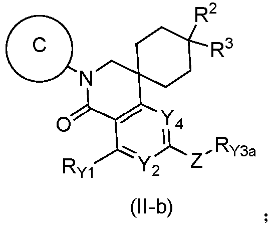
R₂ and R₃ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 R_{Y5aa} groups;
alternatively, R₂, R₃, together with the carbon to which they are attached, form a C₃₋₁₂ cycloalkyl ring or a 3- to 12-membered heterocyclic ring, wherein the C₃₋₁₂ cycloalkyl ring or 3- to 12-membered heterocyclic ring is optionally substituted with 1, 2, 3, or 4 R_{Y5aa} groups;
R_{Y5aa} is independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, and C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group.

8. The compound according to Claims 1 to 5, or a pharmaceutically acceptable salt thereof, wherein the compound has the following structure: 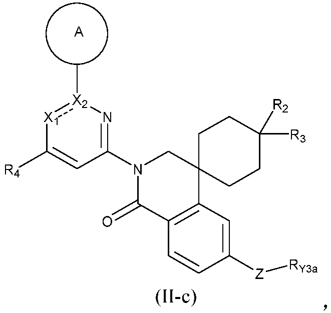
wherein, 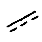 represents a single bond or a double bond;
when 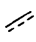 represents a single bond, X₁ is selected from 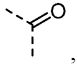 C(Rx₁)₂, and NR_{X1}, and X₂ is selected from N or CR_{X2};
when 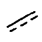 represents a double bond, X₁ is selected from CR_{X1} or N, and X₂ is selected from C;
ring A is selected from a 5- to 12-membered heterocycloalkyl group or a C₃₋₁₂ cycloalkyl group, wherein the 5- to 12-membered heterocycloalkyl group or the C₃₋₁₂ cycloalkyl group is optionally substituted with 1, 2, 3, or 4 R_{A} groups;
R_{X1} and R_{X2} are each independently selected from hydrogen, a halogen, a hydroxyl group, a cyano group, SF₅, a C₁₋₆ alkyl group, a C₂₋₆ alkenyl group, and a C₂₋₆ alkynyl group;
R_{A} is independently selected from hydrogen, a halogen, a hydroxyl group, a cyano group, SF₅, a C₁₋₆ alkyl group, a C₂₋₆ alkenyl group, and a C₂₋₆ alkynyl group;
alternatively, R_{X1} and one of the R_{A} groups, together with the carbon to which they are attached, form a saturated, partially unsaturated, or unsaturated 5- to 9-membered monocyclic ring, wherein the monocyclic ring is optionally substituted with 1, 2, 3, or 4 R_{B} groups;
R_{B} is independently selected from hydrogen, a halogen, a hydroxyl group, a cyano group, SF₅, a C₁₋₆ alkyl group, a C₂₋₆ alkenyl group, and a C₂₋₆ alkynyl group;
R₂, R₃, and R₄ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 R_{Y5aa} groups;
alternatively, R₂, R₃, together with the carbon to which they are attached, form a C₃₋₁₂ cycloalkyl ring or a 5- to 12-membered heterocyclic ring, wherein the C₃₋₁₂ cycloalkyl ring or 5- to 12-membered heterocyclic ring is optionally substituted with 1, 2, 3, or 4 R_{Y5aa} groups;
R_{Y5aa} is independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, and C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group.

9. A compound represented by Formula I, or a pharmaceutically acceptable salt thereof, 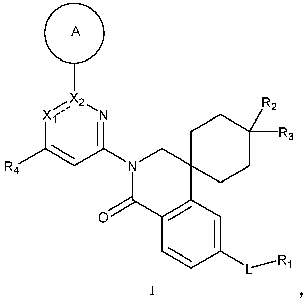
wherein, 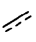 represents a single bond or a double bond;
when 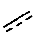 represents a single bond, X₁ is selected from 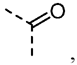 C(R_{X1})₂, and NR_{X1}, and X₂ is selected from N or CR_{X2};
when 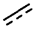 represents a double bond, X₁ is selected from CR_{X1} or N, and X₂ is selected from C;
ring A is selected from a 5- to 6-membered heterocycloalkyl group or a C₅₋₆ cycloalkyl group, wherein the 5- to 6-membered heterocycloalkyl group or the C₅₋₆ cycloalkyl group is optionally substituted with 1, 2, 3, or 4 R_{A} groups;
R_{X1} and R_{X2} are each independently selected from hydrogen, a halogen, a hydroxyl group, a cyano group, SF₅, a C₁₋₆ alkyl group, a C₂₋₆ alkenyl group, and a C₂₋₆ alkynyl group;
R_{A} is independently selected from hydrogen, a halogen, a hydroxyl group, a cyano group, SF₅, a C₁₋₆ alkyl group, a C₂₋₆ alkenyl group, and a C₂₋₆ alkynyl group;
alternatively, R_{X1} and one of the R_{A} groups, together with the carbon to which they are attached, form a saturated, partially unsaturated, or unsaturated 5- to 6-membered monocyclic ring, wherein the monocyclic ring is optionally substituted with 1, 2, 3, or 4 R_{B} groups;
R_{B} is independently selected from hydrogen, a halogen, a hydroxyl group, a cyano group, SF₅, a C₁₋₆ alkyl group, a C₂₋₆ alkenyl group, and a C₂₋₆ alkynyl group;
R₂, R₃, and R₄ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 R_{Y5aa} groups;
alternatively, R₂, R₃, together with the carbon to which they are attached, form a C₃₋₆ cycloalkyl ring or a 3- to 6-membered heterocyclic ring, wherein the C₃₋₆ cycloalkyl ring or 3- to 6-membered heterocyclic ring is optionally substituted with 1, 2, 3, or 4 R_{Y5aa} groups;
R_{Y5aa} is independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, or C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group;
L is selected from a single bond, -C₀₋₄ alkyl-S-C₀₋₄ alkyl-, -C₀₋₄ alkyl-S(=O)-C₀₋₄ alkyl-, -C₀₋₄ alkylS(=O)₂-C₀₋₄ alkyl-, -C₀₋₄ alkyl-NR_{Y3b}-C₀₋₄ alkyl-, -C₀₋₄ alkyl-NR_{Y3b}-S(=O)₂-C₀₋₄ alkyl-, -C₀₋₄ alkyl-S(=O)₂-NR_{Y3b}-₀₋₄ alkyl-, -C₀₋₄ alkyl-O-C₀₋₄ alkyl-, -C₀₋₄ alkyl-C(=O)-C₀₋₄ alkyl-, -C₀₋₄ alkyl-C(=O)-O-C₀₋₄ alkyl-, - C₀₋₄ alkyl-C(=O)-NR_{Y3b}-C₀₋₄ alkyl-, -C₀₋₄ alkyl-S(=O)(=NH)-, -N=S(=O)-, -(C=O)-, or -C(=N-OH)-;
R_{Y3b} is independently selected from hydrogen, a halogen, a hydroxyl group, a cyano group, SF₅, and a C₁₋₆ alkyl group;
R₁ is independently selected from hydrogen, a halogen, a hydroxyl group, a cyano group, SF₅, a C₁₋₆ alkyl group, a C₃₋₆ cycloalkyl group, and a 5-6 membered heterocyclyl group, wherein the C₁₋₆ alkyl group, C₃₋₆ cycloalkyl group, or 5-6 membered heterocyclyl group is optionally substituted with 1, 2, 3, or 4 R' groups;
R' is independently selected from hydrogen, a halogen, a hydroxyl group, a cyano group, SF₅, and a C₁₋₆ alkyl group;
alternatively, two R' groups are attached to the same carbon atom to form =C(F)₂ or =O.

10. A compound represented by Formula I, or a pharmaceutically acceptable salt thereof, 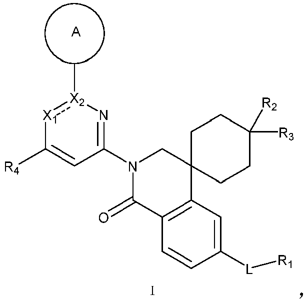 wherein: 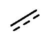
represents a single bond or a double bond;
when 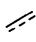 represents a single bond, X₁ is selected from 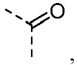 and X₂ is selected from N;
when 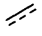 represents a double bond, X₁ is selected from CH or N, and X₂ is selected from C;
ring A is selected from a 4- to 6-membered heterocycloalkyl group or a C₃₋₆ cycloalkyl group, wherein the 4- to 6-membered heterocycloalkyl group or the C₃₋₆ cycloalkyl group is optionally substituted with 1 or 2 groups each independently selected from a halogen or a C₁₋₃ alkyl group;
-L-R₁ is selected from 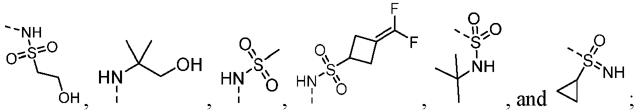
R₂, R₃, and R₄ are each independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, and C₂₋₆ alkynyl group is optionally substituted with 1, 2, or 3 R_{Y5aa} groups;
alternatively, R₂, R₃, together with the carbon to which they are attached, form a C₃₋₆ cycloalkyl ring or a 5- to 6-membered heterocyclic ring, wherein the C₃₋₆ cycloalkyl ring or 5- to 6-membered heterocyclic ring is optionally substituted with 1, 2, 3, or 4 R_{Y5aa} groups;
R_{Y5aa} is independently selected from hydrogen, a halogen, a hydroxyl group, a cyano group, SF₅, a C₁₋₆ alkyl group, a C₂₋₆ alkenyl group, and a C₂₋₆ alkynyl group, wherein the C₁₋₆ alkyl group, C₂₋₆ alkenyl group, and C₂₋₆ alkynyl group is optionally substituted with 1, 2, or 3 groups independently selected from hydrogen, a halogen, a hydroxyl group, a cyano group, SF₅, a C₁₋₆ alkyl group, a C₂₋₆ alkenyl group, and a C₂₋₆ alkynyl group.

11. A compound represented by Formula I, or a pharmaceutically acceptable salt thereof, 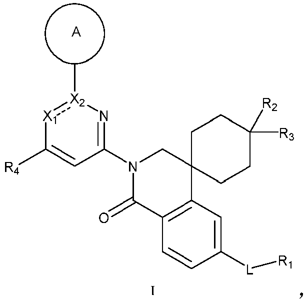 wherein: 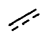
represents a single bond or a double bond;
when 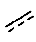 represents a single bond, X₁ is selected from 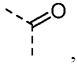 and X₂ is selected from N;
when 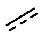 represents a double bond, X₁ is selected from CH or N, and X₂ is selected from C;
ring A is selected from a 6-membered heterocycloalkyl group or a cyclobutyl group, wherein the 6-membered heterocycloalkyl group or the cyclobutyl group is optionally substituted with 1 or 2 groups each independently selected from a halogen or a C₁₋₃ alkyl group;
L is selected from NH;
R₁ is selected from -S(=O)₂-C₁₋₃ alkyl-OH;
R₂ and R₃ are each independently selected from H or a C₁₋₃ alkyl group;
alternatively, R₂, R₃, and the carbon to which they are attached together form a cyclopropyl ring;
R₄ is selected from H or a C₁₋₃ alkyl group.

12. The compound according to any one of Claims 9 to 11, or a pharmaceutically acceptable salt thereof, wherein ring A is selected from a morpholinyl group, a piperidinyl group, or a cyclobutyl group, wherein the morpholinyl group, piperidinyl group, or cyclobutyl group is optionally substituted with 1 or 2 groups independently selected from F or a C₁₋₃ alkyl group.

13. The compound according to any one of Claims 9 to **11,** or a pharmaceutically acceptable salt thereof, wherein ring A is selected from

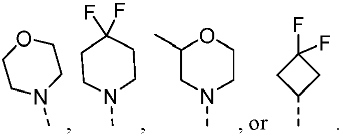

14. The compound according to any one of Claims 9 to 11, or a pharmaceutically acceptable salt thereof, wherein R₄ is selected from H or a methyl group.

15. The compound according to any one of Claims 9 to 11, or a pharmaceutically acceptable salt thereof, wherein, R₂ and R₃ are each independently selected from H or a methyl group.

16. The compound according to any one of Claims 9 to 11, or a pharmaceutically acceptable salt thereof, wherein R₁ is selected from

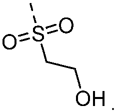

17. The compound according to any one of Claims 1 to 11, or a pharmaceutically acceptable salt thereof, wherein the compound is selected from one of the following structures:

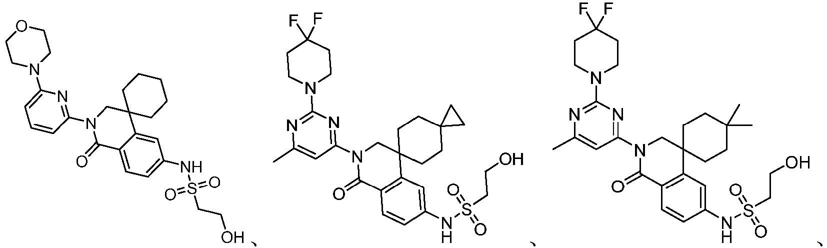

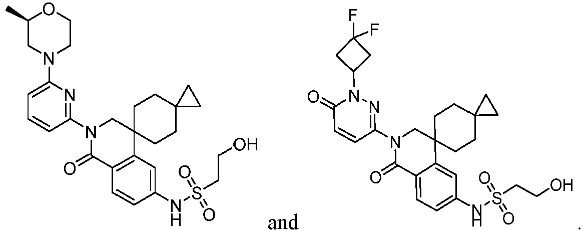

18. The compound according to Claim 1 or a pharmaceutically acceptable salt thereof, wherein the compound is selected from one of those listed in Table a.

19. A pharmaceutical composition, comprising a compound according to any one of Claims 1 to 18, or a pharmaceutically acceptable salt thereof,
and a pharmaceutically acceptable carrier or excipient.

20. The use of a substance X in the preparation of a KIF18A inhibitor, wherein the substance X is a compound according to any one of Claims 1 to 18, or a pharmaceutically acceptable salt thereof, or the pharmaceutical composition according to Claim 19.

21. The use of a substance X in the preparation of a medicament, wherein the substance X is a compound according to any one of Claims 1 to 18, or a pharmaceutically acceptable salt thereof, or the pharmaceutical composition according to Claim 19;
wherein the medicament is for the treatment of cancer.
